# EUROPEAN PATENT SPECIFICATION

(11) **EP 3 559 674 B1**
(45) Date of publication and mention of the grant of the patent: **07.07.2021**
(21) Application number: 17838039.0
(22) Date of filing: 19.12.2017
(51) Int. Cl.: G01N 33/68

(54) **A METHOD TO DETERMINE BRAF MUTATIONS AND WILD TYPE BRAF PROTEIN BY MASS SPECTROMETRY**
VERFAHREN ZUR BESTIMMUNG VON BRAF-MUTATIONEN UND WILDTYP-BRAF-PROTEIN DURCH MASSENSPEKTROMETRIE
PROCÉDÉ PERMETTANT DE DÉTERMINER DES MUTATIONS DE BRAF ET UNE PROTÉINE BRAF DE TYPE SAUVAGE PAR SPECTROMÉTRIE DE MASSE

(30) Priority: 20.12.2016 SE 1651685
(43) Date of publication of application: 30.10.2019
(73) Proprietor: Treat4Life AB, 212 14 Malmö (SE)
(72) Inventor: MARKO-VARGA, Gyorgy, 211 49 Malmö (SE); WELINDER, Charlotte, 243 34 Hörby (SE); SUGIHARA, Yutaka, 212 17 Malmö (SE)
(74) Representative: Ström & Gulliksson AB
(86) International application number: PCT/EP2017/083547
(87) International publication number: WO 2018/114953

(56) References cited:
- US-A1- 2015 344 957
- US-A1- 2018 017 549
- CHEN HANG ET AL: "Quantitative analysis of wild-type and V600E mutant BRAF proteins in colorectal carcinoma using immunoenrichment and targeted mass spectrometry", ANALYTICA CHIMICA ACTA, ELSEVIER, AMSTERDAM, NL, vol. 933, 2 June 2016 (2016-06-02), pages 144-155, XP029672879, ISSN: 0003-2670, DOI: 10.1016/J.ACA.2016.05.037
- MARK KRIEGSMANN ET AL: "Detection of KRAS, NRAS and BRAF by mass spectrometry - a sensitive, reliable, fast and cost-effective technique", DIAGNOSTIC PATHOLOGY, BIOMED CENTRAL LTD, LO, vol. 10, no. 1, 30 July 2015 (2015-07-30), page 132, XP021228260, ISSN: 1746-1596, DOI: 10.1186/S13000-015-0364-3
- PAOLO A ASCIERTO ET AL: "The role of BRAF V600 mutation in melanoma", JOURNAL OF TRANSLATIONAL MEDICINE, BIOMED CENTRAL, vol. 10, no. 1, 9 July 2012 (2012-07-09), page 85, XP021126777, ISSN: 1479-5876, DOI: 10.1186/1479-5876-10-85
- BISHU SAPKOTA ET AL: "Vemurafenib enhances MHC induction in BRAF V600E homozygous melanoma cells", ONCOIMMUNOLOGY, vol. 2, no. 1, 1 January 2013 (2013-01-01) , page e22890, XP055459770, DOI: 10.4161/onci.22890 cited in the application

### SEQUENCE LISTING

<110> TREAT4LIFE AB
<120> A METHOD TO DETERMINE BRAF MUTATIONS AND WILD TYPE BRAF PROTEIN BY MASS SPECTROMETRY
<130> W133380002
<150> SE 1651685-8
   <151> 2016-12-20
<160> 45
<170> PatentIn version 3.5
<210> 1
   <211> 25
   <212> PRT
   <213> Homo sapiens
<400> 1
   Asp Leu Thr Val Lys Ile Gly Asp Phe Gly Leu Ala Thr Val Lys Ser
   1               5                   10                  15
   Arg Trp Ser Gly Ser His Gln Phe Glu
               20                  25
<210> 2
   <211> 14
   <212> PRT
   <213> Homo sapiens
<400> 2
   Asp Leu Thr Val Lys Ile Gly Asp Phe Gly Leu Ala Thr Glu
   1 5 10
<210> 3
   <211> 25
   <212> PRT
   <213> Homo sapiens
<400> 3
   Asp Leu Thr Val Lys Ile Gly Asp Phe Gly Leu Ala Thr Lys Lys Ser
   1 5 10 15
   Arg Trp Ser Gly Ser His Gln Phe Glu
   20 25
<210> 4
   <211> 25
   <212> PRT
   <213> Homo sapiens
<400> 4
   Asp Leu Thr Val Lys Ile Gly Asp Phe Gly Leu Ala Thr Arg Lys Ser
   1 5 10 15
   Arg Trp Ser Gly Ser His Gln Phe Glu
   20 25
<210> 5
   <211> 25
   <212> PRT
   <213> Homo sapiens
<400> 5
   Asp Leu Thr Val Lys Ile Gly Asp Phe Gly Leu Ala Thr Asp Lys Ser
   1 5 10 15
   Arg Trp Ser Gly Ser His Gln Phe Glu
   20 25
<210> 6
   <211> 6
   <212> **PRT**
   <213> Homo sapiens
<400> 6
   Phe Gly Leu Ala Thr Glu
   1 5
<210> 7
   <211> 5
   <212> **PRT**
   <213> Homo sapiens
<400> 7
   Gly Leu Ala Thr Asp
   1               5
<210> 8
   <211> 17
   <212> **PRT**
   <213> Homo sapiens
<400> 8
   Phe Gly Leu Ala Thr Val Lys Ser Arg Trp Ser Gly Ser His Gln Phe
   1               5                   10                  15
   Glu
<210> 9
   <211> 17
   <212> PRT
   <213> Homo sapiens
<400> 9
   Phe Gly Leu Ala Thr Lys Lys Ser Arg Trp Ser Gly Ser His Gln Phe
   1               5                   10                  15
   Glu
<210> 10
   <211> 17
   <212> PRT
   <213> Homo sapiens
<400> 10
   Phe Gly Leu Ala Thr Arg Lys Ser Arg Trp Ser Gly Ser His Gln Phe
   1               5                   10                  15
   Glu
<210> 11
   <211> 24
   <212> PRT
   <213> Homo sapiens
<400> 11
   Leu Thr Val Lys Ile Gly Asp Phe Gly Leu Ala Thr Val Lys Ser Arg
   1               5                   10                  15
   Trp Ser Gly Ser His Gln Phe Glu
               20
<210> 12
   <211> 18
   <212> PRT
   <213> Homo sapiens
<400> 12
   Asp Phe Gly Leu Ala Thr Val Lys Ser Arg Trp Ser Gly Ser His Gln
   1               5                   10                  15
   Phe Glu
<210> 13
   <211> 25
   <212> PRT
   <213> Homo sapiens
<400> 13
   Asp Leu Thr Val Lys Ile Gly Asp Phe Gly Leu Ala Thr Glu Lys Ser
   1               5                   10                  15
   Arg Trp Ser Gly Ser His Gln Phe Glu
               20                  25
<210> 14
   <211> 18
   <212> PRT
   <213> Homo sapiens
<400> 14
   Asp Phe Gly Leu Ala Thr Glu Lys Ser Arg Trp Ser Gly Ser His Gln
   1               5                   10                  15
   Phe Glu
<210> 15
   <211> 17
   <212> PRT
   <213> Homo sapiens
<400> 15
   Phe Gly Leu Ala Thr Glu Lys Ser Arg Trp Ser Gly Ser His Gln Phe
   1               5                   10                  15
   Glu
<210> 16
   <211> 24
   <212> **PRT**
   <213> Homo sapiens
<400> 16
   Leu Thr Val Lys Ile Gly Asp Phe Gly Leu Ala Thr Glu Lys Ser Arg
   1               5                   10                  15
   Trp Ser Gly Ser His Gln Phe Glu
               20
<210> 17
   <211> 25
   <212> PRT
   <213> Homo sapiens
<400> 17
   Asp Leu Thr Val Lys Ile Gly Asp Phe Gly Leu Ala Thr Glu Lys Ser
     1               5                   10                  15
   Arg Trp Ser Gly Ser His Gln Phe Glu
               20                  25
<210> 18
   <211> 24
   <212> PRT
   <213> Homo sapiens
<400> 18
   Leu Thr Val Lys Ile Gly Asp Phe Gly Leu Ala Thr Glu Lys Ser Arg
   1               5                   10                  15
   Trp Ser Gly Ser His Gln Phe Glu
               20
<210> 19
   <211> 18
   <212> PRT
   <213> Homo sapiens
<400> 19
   Asp Phe Gly Leu Ala Thr Glu Lys Ser Arg Trp Ser Gly Ser His Gln
   1               5                   10                  15
   Phe Glu
<210> 20
   <211> 17
   <212> PRT
   <213> Homo sapiens
<400> 20
   Phe Gly Leu Ala Thr Glu Lys Ser Arg Trp Ser Gly Ser His Gln Phe
   1               5                   10                  15
   Glu
<210> 21
   <211> 13
   <212> PRT
   <213> Homo sapiens
<400> 21
   Leu Thr Val Lys Ile Gly Asp Phe Gly Leu Ala Thr Glu
   1               5                   10
<210> 22
   <211> 7
   <212> PRT
   <213> Homo sapiens
<400> 22
   Asp Phe Gly Leu Ala Thr Glu
   1               5
<210> 23
   <211> 18
   <212> PRT
   <213> Homo sapiens
<400> 23
   Asp Phe Gly Leu Ala Thr Lys Lys Ser Arg Trp Ser Gly Ser His Gln
   1               5                   10                  15
   Phe Glu
<210> 24
   <211> 17
   <212> PRT
   <213> Homo sapiens
<400> 24
   Phe Gly Leu Ala Thr Lys Lys Ser Arg Trp Ser Gly Ser His Gln Phe
   1               5                   10                  15
   Glu
<210> 25
   <211> 24
   <212> PRT
   <213> Homo sapiens
<400> 25
   Leu Thr Val Lys Ile Gly Asp Phe Gly Leu Ala Thr Lys Lys Ser Arg
   1               5                   10                  15
   Trp Ser Gly Ser His Gln Phe Glu
               20
<210> 26
   <211> 24
   <212> PRT
   <213> Homo sapiens
<400> 26
   Asp Leu Thr Val Lys Ile Gly Asp Phe Gly Leu Ala Thr Lys Ser Arg
   1               5                   10                  15
   Trp Ser Gly Ser His Gln Phe Glu
               20
<210> 27
   <211> 24
   <212> PRT
   <213> Homo sapiens
<400> 27
   Leu Thr Val Lys Ile Gly Asp Phe Gly Leu Ala Thr Lys Lys Ser Arg
   1               5                   10                  15
   Trp Ser Gly Ser His Gln Phe Glu
               20
<210> 28
   <211> 18
   <212> PRT
   <213> Homo sapiens
<400> 28
   Asp Phe Gly Leu Ala Thr Lys Lys Ser Arg Trp Ser Gly Ser His Gln
   1 5 10 15
   Phe Glu
<210> 29
   <211> 18
   <212> PRT
   <213> Homo sapiens
<400> 29
   Asp Phe Gly Leu Ala Thr Arg Lys Ser Arg Trp Ser Gly Ser His Gln
   1 5 10 15
   Phe Glu
<210> 30
   <211> 17
   <212> PRT
   <213> Homo sapiens
<400> 30
   Phe Gly Leu Ala Thr Arg Lys Ser Arg Trp Ser Gly Ser His Gln Phe
   1 5 10 15
   Glu
<210> 31
   <211> 24
   <212> PRT
   <213> Homo sapiens
<400> 31
   Leu Thr Val Lys Ile Gly Asp Phe Gly Leu Ala Thr Arg Lys Ser Arg
   1 5 10 15
   Trp Ser Gly Ser His Gln Phe Glu
   20
<210> 32
   <211> 25
   <212> PRT
   <213> Homo sapiens
<400> 32
   Asp Leu Thr Val Lys Ile Gly Asp Phe Gly Leu Ala Thr Arg Lys Ser
   1               5                   10                  15
   Arg Trp Ser Gly Ser His Gln Phe Glu
               20                  25
<210> 33
   <211> 24
   <212> PRT
   <213> Homo sapiens
<400> 33
   Leu Thr Val Lys Ile Gly Asp Phe Gly Leu Ala Thr Arg Lys Ser Arg
   1               5                   10                  15
   Trp Ser Gly Ser His Gln Phe Glu
               20
<210> 34
   <211> 18
   <212> PRT
   <213> Homo sapiens
<400> 34
   Asp Phe Gly Leu Ala Thr Arg Lys Ser Arg Trp Ser Gly Ser His Gln
   1               5                   10                  15
   Phe Glu
<210> 35
   <211> 18
   <212> PRT
   <213> Homo sapiens
<400> 35
   Asp Phe Gly Leu Ala Thr Asp Lys Ser Arg Trp Ser Gly Ser His Gln
   1               5                   10                  15
   Phe Glu
<210> 36
   <211> 17
   <212> PRT
   <213> Homo sapiens
<400> 36
   Phe Gly Leu Ala Thr Asp Lys Ser Arg Trp Ser Gly Ser His Gln Phe
   1               5                   10                  15
   Glu
<210> 37
   <211> 24
   <212> PRT
   <213> Homo sapiens
<400> 37
   Leu Thr Val Lys Ile Gly Asp Phe Gly Leu Ala Thr Asp Lys Ser Arg
   1               5                   10                  15
   Trp Ser Gly Ser His Gln Phe Glu
               20
<210> 38
   <211> 25
   <212> PRT
   <213> Homo sapiens
<400> 38
   Asp Leu Thr Val Lys Ile Gly Asp Phe Gly Leu Ala Thr Asp Lys Ser
   1               5                   10                  15
   Arg Trp Ser Gly Ser His Gln Phe Glu
               20                  25
<210> 39
   <211> 24
   <212> PRT
   <213> Homo sapiens
<400> 39
   Leu Thr Val Lys Ile Gly Asp Phe Gly Leu Ala Thr Asp Lys Ser Arg
   1               5                   10                  15
   Trp Ser Gly Ser His Gln Phe Glu
               20
<210> 40
   <211> 18
   <212> PRT
   <213> Homo sapiens
<400> 40
   Asp Phe Gly Leu Ala Thr Asp Lys Ser Arg Trp Ser Gly Ser His Gln
   1 5 10 15
   Phe Glu
<210> 41
   <211> 17
   <212> PRT
   <213> Homo sapiens
<400> 41
   Phe Gly Leu Ala Thr Asp Lys Ser Arg Trp Ser Gly Ser His Gln Phe
   1 5 10 15
   Glu
<210> 42
   <211> 14
   <212> PRT
   <213> Homo sapiens
<400> 42
   Asp Leu Thr Val Lys Ile Gly Asp Phe Gly Leu Ala Thr Asp
   1 5 10
<210> 43
   <211> 13
   <212> PRT
   <213> Homo sapiens
<400> 43
   Leu Thr Val Lys Ile Gly Asp Phe Gly Leu Ala Thr Asp
   1 5 10
<210> 44
   <211> 7
   <212> PRT
   <213> Homo sapiens
<400> 44
   Asp Phe Gly Leu Ala Thr Asp
   1 5
<210> 45
   <211> 6
   <212> PRT
   <213> Homo sapiens
<400> 45
   Phe Gly Leu Ala Thr Asp
   1               5

## Description

### Field of the Invention

This invention pertains in general to the field of medical diagnosis and targeted therapy. More particularly the invention relates to a specific method for the determination of amino acid speciation of the Serine/Threonine Protein Kinase BRAF sequences; wild type and mutated forms by mass spectrometry methodology. More particularly the method relates to the ability to analyze the BRAF sequences with a point mutation, such as amino acid position 600. Furthermore, the present invention pertains to provide a clinical utility for making decision for personalized treatment of melanoma patients with kinase inhibitors.

### Background of the Invention

It is known that the ever increasing demands in cancer healthcare today posture high forecasts and directions onto the research community to develop new solutions that can improve clinical outcome with improved cost efficiency. In reply to these challenges, modern healthcare is looking for ways to treat patients that are both more efficient and beneficial for the patient, as well as more cost saving. The outline of regulatory directives are vital to our community to manage and meet the demands from cancer patients that are expecting drugs that are more safe, with lower mortalities, and with a fast onset of efficacy.

The National Cancer Institute a part of National Institutes of Health and local clinicians and scientists has made an extensive progress to work out and provide a protein biomarker discovery and validation strategy. These regulatory guidelines provide high-quality standardized, sensitive, specific, quantitative, and readily accessible protein, peptide, or other biomarkers of health, disease, response to therapy into the approval processes of regulatory agencies (e.g., U.S. Food and Drug Administration; FDA).

It is a well-known fact that the cause of about 22% of cancer deaths is due to tobacco use. Another 10% is due to overweight and obesity, a poor diet, and often a lack of physical exercise and over consumption of alcohol. Other factors include certain infections and/or exposure to infections and environmental pollutants. In the developing world close to 20% of cancers are due to infections such as Hepatitis B, Hepatitis C and human papilloma virus. These factors act, at least partly, by changing the genes and proteins of a cell. Typically many such genetic changes are required before cancer develops. Statistically, approximately 5-10% of cancers are due to genetic defects inherited from a person's parents.

Personalized medicine is a medical treatment model, tailored to the individual patient. Within Personalized Medicine optimal therapies are often employed for selecting appropriate treatments based on the context of a patient's genetic content or other molecular or cellular analysis. The use of genetic information, pharmacogenomics, has played a major role in certain aspects of personalized medicine, and the term was first coined in the context of genetics, though it has since broadened to encompass all sorts of targeted personal read out diagnosis testing.

Screening and measuring each and every patient on an individual basis will allow for a more exact diagnosis and personalized action plan. Modern genotyping provides a detailed account of an individual's DNA sequence; their genome can then be compared to a reference genome, in order to assess the existing genetic variations that can account for conceivable disease status.

A precise treatment, by targeted therapy, using "Personalized Medicine" is able to greatly aid in the advancements of preventive care. This has been proven over the years, where women are being genotyped for certain mutations in the BRCA1 and BRCA2 genes, respectively, investigating predisposition because of a family history of melanoma, breast-, and/or ovarian cancer.

By the identity of a multitude of sources of disease presentation, which are mapped out according to mutations that exist within a genome, indicate that the easier they can be identified in an individual, the better opportunity for successful treatments. Companion diagnostics is the definition that is being used to test efficacy and safety of a drug specific to a targeted patient group or sub-group. In many instances the companion diagnostics assay is accommodating in enhancing the therapeutic treatment effectiveness.

Today, in modern healthcare, it is communal that physicians often use a trial and error procedure, until they find the treatment therapy that is most effective for their patient. Hence, improved treatment methods taking the individual into account, would be advantageous with novel methods that could aid in the guidance of treatment in the highly complex disease biology.

The Mitogen-Activated Protein Kinase (MAPK) signaling and the corresponding pathway activation, plays a central role in cellular growth, differentiation, and stress response. Within cancer diseases, the MAPK pathway activity has been implicated in many types of cancer developments. This pathway is in general activated by the binding of extracellular growth factors to membrane-bound receptors, which then recruit intracellular proteins to the cell membrane, leading to the activation of the small guanosine triphosphate-binding protein, RAS. Thus, RAS adopts an activated conformation that stimulates the signaling downstream. This will result in a phosphorylation and activation of ERK, which controls a wide range of functional processes within the cell. This pathway can also be activated by the mutation of specific proteins, including BRAF. Such activating mutations appear to mimic regulatory phosphorylation of BRAF and increase its kinase activity compared with the wild-type protein.

Malignant melanoma is the sixth most common cancer worldwide with an increasing incidence in the Northern European countries and Australia. According to World Health Organization, there are about 132 000 new cases of melanoma diagnosed worldwide each year. The majority of early cases of melanoma are cured surgically; however some primary tumors will relapse and become metastatic. The American Joint Committee on Cancer (AJCC) staging of the tumors is based on tumor thickness, mitotic rate and ulceration as well as on regional and distant spread. Malignant melanoma has been inherently difficult to treat with a very low 5 year survival (< 15%).

BRAF mutations that constitutively trigger MAPK signaling and bypass the need for upstream stimuli happen with high incidence in malignant melanoma. Correspondingly, the cellular inhibition of BRAFV600E kinase activity by drug impact will result in a decreased MEK and ERK phosphorylation. Approximately 90% of all BRAF mutations identified in human cancers are a T1799A transversion in exon 15, which results in a V600E amino acid substitution and BRAF kinase activation. The high frequency of activating mutations in tumors and ensuing MAPK pathway addiction make BRAF an attractive therapeutic target, where inhibition of the kinase activity of BRAFV600E and other activated BRAF mutants could provide an effective therapy.

BRAF inhibitors with diverse levels of selectivity have been identified and clinically tested. The cancer spread and initiation phases of disease can be detected by certain signs and symptoms or diagnostic tests. It is then typically further investigated by various types of medical imaging platforms, such as X-ray computed tomography (CT), positron emission tomography (PET), magnetic resonance imaging (MRI) or mass spectrometry imaging and confirmed by pathology diagnosis of a biopsy. The benefits of screening in breast cancer are valuable both for the patient as well as our society, as proven by the treatment of woman and breast cancer as well as for men with prostate cancer where in both diseases the diagnosis development has decreased the mortality rates significantly over the last decade.

The most common cancer types in males are lung cancer, prostate cancer, colorectal cancer and stomach cancer. The most frequent events in woman are breast cancer, colorectal cancer, lung cancer and cervical cancer. However, if one would include skin and all types of malignant melanoma, this would account for about 40% of the cases. With respect to children, the cancer types vary, and the most common are acute lymphoblastic leukemia and brain tumors. In Africa however, the most common cancer disease is Non-Hodgkin's lymphoma. The financial costs of cancer have been estimated at $1.16 trillion US dollars per year as of 2010.

Most melanomas harbor alterations in the BRAF, NP1, RAS, MDM2 (KIT) genes, which result in activation of the MAPK and RAS pathways conferring survival advantage by reprogramming crucial cell cycle and apoptotic cellular functions. Clinical and pathological properties partly reflect the identified (BRAF, RAS, NF1, triple wild type) genomic subtypes of melanoma, but from both clinical and genetic perspective the groups still are heterogeneous.

Newly developed drugs allowing targeted therapy such as kinase inhibitors or drugs modulating the immune response provide more promise. Two compounds Vemurafenib and Dabrafenib (kinase inhibitors) have achieved approval by FDA for the treatment of metastatic and unresectable BRAF-mutated (V600E) melanomas. Trametinib, a mitogen-activated extracellular signal regulated kinase (MEK) inhibitor, is also FDA-approved. However, these newer treatments have also been subjected to resistance development. Recently, FDA approved Nivolumab (anti-PDl antibody) in combination with Ipilimumab (anti-CTLA-4 antibody), for the treatment of patients both with BRAF V600 wild-type and BRAF mutation unresectable metastatic melanoma.

In Chen Hang et.al., Anal. Chim. Acta, vol. 933 (2016), p. 144-155, immuno-enrichment coupled with two MS-based quantitative techniques are evaluated to detect and quantify wild-type (WT) and V600E mutant BRAF proteins in DNA sequence-confirmed CRC tissue specimens.

In Mark Kriegsmann et.al., Diagn. Pathol. (2015), 10:132, an assay for routine mass-spectrometric analysis to test the presence/absence of 18 KRAS, 14 NRAS and 4 BRAF mutations on a DNA level is disclosed.

In US 2015/344957 A1 methods of determining the amount of a BRAF V600E mutation on a DNA level over time in a subject with Langerhans Cell Histiocytosis (LCH) or Erdheim-Chester Disease (ECD) who is being treated with vemurafenib or dabrafenib are disclosed.

In Paolo A Ascierto et.al., J. Transl. Med., (2012), 10:85, it is suggested to use vemurafenib and dabrafenib targeting BRAF mutations for treatment of melanoma.

Thus, there is a great demand to investigate *BRAF* mutations and the consequences of these on an individual basis, before starting dedicated treatment of malignant melanoma patients with targeted kinase inhibitor medicines.

### Summary of the Invention

Accordingly, the present invention preferably seeks to mitigate, alleviate or eliminate one or more of the above-identified deficiencies in the art and disadvantages singly or in any combination and solves at least the above mentioned problems by providing a method for determining the molar ratio between wildtype (WT) BRAF protein and protein variants thereof in a biological sample, wherein the BRAF protein variants are variants mutated in the position corresponding to amino acid position 600 in WT BRAF, said position being occupied by valine in the WT BRAF, comprising the steps of: a) Digesting said sample by using a serine proteinase which specifically cleaves peptide bonds C-terminal to glutamic acid residues or peptide bonds C-terminal to glutamic or aspartic acid residues, to obtain a composition comprising a peptide fragment resulting from digestion of the peptides by the proteinase, wherein the mass of said fragment differs between said wildtype (WT) B-raf protein and a BRAF protein variant. b) Quantitatively assaying the molar amount of a peptide fragment resulting from digestion of wildtype (WT) B-raf protein and the molar amount of the peptide fragment resulting from digestion of variants of the wildtype (WT) B-raf protein using a mass spectrometry technique. And, c) based on the quantitative assessment calculating the at least one specific ratio between said WT BRAF protein and a variant thereof.

Also provided is a method for estimating a subject's susceptibility to a given drug treatment for a BRAF related disease, comprising the steps of (i) providing a sample from a subject suffering from a BRAF related disease; and determining the specific molar ratio between WT BRAF protein and variants thereof by a method according to any of claims 1 to 9. (ii) comparing the molar specific ratio between WT BRAF protein and variants thereof, with a reference value of the specific molar ratio between WT BRAF protein and variant thereof determined from a multitude of samples from subjects known to suffer from said BRAF related disease and to be susceptible to said given drug treatment. (iii) based on said comparison determining the subject's susceptibility to a given drug treatment, wherein a ratio defined by WT BRAF protein to said at least one BRAF protein variant in said sample above the ratio defined by WT BRAF protein to said at least one BRAF protein variant reference value is indicative for an increased susceptibility to a given drug treatment, the reference value is a specific molar ratio between WT BRAF protein and said at least one BRAF protein variant determined from a multitude of samples from subjects known to suffer from said BRAF related disease and to be susceptible to said given drug treatment, the BRAF-related disease is a cancer with BRAF mutations at amino acid position 600, and said drug treatment is a treatment using a kinase inhibitor, such as Vemurafenib, Dabrafenib or Sorafenib.

Further provided, but not falling under the claims, is a method of treatment for a subject with a BRAF related disease, comprises the steps of providing (i) providing a sample from a subject suffering from a BRAF related disease; and determining the specific molar ratio between WT BRAF protein and variants thereof by a method according to any of claims 1 to 9. (ii) comparing the molar specific ratio between WT BRAF protein and variants thereof, with a reference value of the specific molar ratio between WT BRAF protein and variant thereof determined from a multitude of samples from subjects known to suffer from said BRAF related disease and to be susceptible to said given drug treatment. (iii) based on said comparison determining the subject's susceptibility to a given drug treatment, wherein a ratio or WT BRAF to said at least one BRAF protein variant in said sample above the ratio or WT BRAF to said at least one BRAF protein variant reference value is indicative for an increased susceptibility to a given drug treatment. And, (iv) if the patient is found susceptibility to a given drug treatment, administer the drug of said drug treatment at a prescribed or defined daily dose (DDD) for a prescribed treatment period, or if the patient if found not to be susceptibility to a given drug treatment, start alternative treatments instead, such as surgery, radiation therapy, chemotherapy, immunotherapy and/or other treatments beneficial for said BRAF related disease.

### Brief Description of the Drawings

These and other aspects, features and advantages of which the invention is capable of will be apparent and elucidated from the following description of embodiments of the present invention, reference being made to the accompanying drawings, in which;

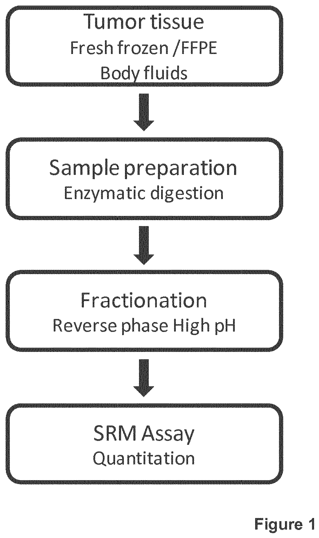

Fig. 1 is an illustration of the general experimental procedure of SRM-based quantification of BRAF (wild type and mutated forms),
Fig. 2 demonstrates the SRM signature precursor chromatographic peaks for the GluC generated peptides, the wild type and the four mutated variants of BRAF, in tumor tissue lysate from a fresh frozen tumor sample. The retention times for WT, V600E, V600D, V600 R and V600K are 19.9, 23.2, 18,3, 15.9 and 15.8 min, respectively,
Fig. 3 shows the characteristic transition ions that define the SRM signature of the peptides in tumor tissue lysate from a fresh frozen tumor sample. Here two examples are shown A) wild type and B) V600E, and
Fig. 4 illustrates one of the BRAF mutations at amino acid position 600 (V600E) and the cellular inhibition of BRAFV600E kinase activity resulting in a decreased MEK phosphorylation.

### Description of embodiments

Lately, the cancer field has discovered a great deal about the genetic variety of cancer types that is presented within traditional disease pathology. The definition of the tumor heterogeneity among cancer patients is a genetic diversity within a single tumor. Among other prospects, these discoveries raise the possibility of identifying, that drugs with poor outcome applied to a general population, may yet be successful for a proportion of cases with a particular genetic profile.

With personalized medicine, treatments can be more specifically tailored to an individual and give insight into how their body will respond to the drug and if that drug will work based on their genome, and subsequent transcript with a final expressed protein. Genotyping is a valued tool for personalized medicine, where differences in the genetic make-up (genotype) of an individual are determined, by examining the individual's DNA sequence and comparing it to a reference sequence. Although this can discover the presence of gene mutations in an individual, it is known that gene expression most often does not correspond with the protein level of the cell. Furthermore, there is the possibility of having multiple genes present, expressing both mutated and wildtype protein.

Phenotyping made by protein sequencing is the only way that the true mutation and wild type status can be assured on a quantitative level, providing a stoichiometry measurement of non-mutated and mutated forms of BRAF

The Mitogen-Activated Protein Kinase (MAPK) signaling and the corresponding pathway activation, plays a central role in cellular growth, differentiation, and stress response. Within cancer diseases, the MAPK pathway activity has been implicated in many types of cancer developments. This pathway is in general activated by the binding of extracellular growth factors to membrane-bound receptors, which then recruit intracellular proteins to the cell membrane, leading to the activation of the small guanosine triphosphate-binding protein, RAS. Thus, RAS adopts an activated conformation that stimulates the signaling downstream. This will result in a phosphorylation and activation of ERK, which controls a wide range of functional processes within the cell. This pathway can also be activated by the mutation of specific proteins, including BRAF. Such activating mutations appear to mimic regulatory phosphorylation of BRAF and increase its kinase activity compared with the wild-type protein.

BRAF mutations that constitutively trigger MAPK signaling and bypass the need for upstream stimuli happen with high incidence in malignant melanoma. Correspondingly, the cellular inhibition of BRAFV600E kinase activity by drug impact will result in a decreased MEK and ERK phosphorylation. The functional effect of these post-translational modifications, will result in an inhibition of cell proliferation through an initial G1 cell cycle arrest, followed by cell death.

So far, more than 45 cancer-associated BRAF mutations have been identified with a high frequency in specific cancers, where 40-60% are linked to malignant melanoma. Approximately 90% of all BRAF mutations identified in human cancers are a T1799A transversion in exon 15, which results in a V600E amino acid substitution and BRAF kinase activation. It has been reported that the mutation variation is >80% in melanomas cancers and nevi (sharply circumscribed and chronic lesions of the skin or mucosa). It has also been reported a lower range of incidences; ranging from 0 to 18% in other tumors (Namba H, Nakashima M, Hayashi T, Hayashida N, Maeda S, Rogounovitch TI, Ohtsuru A, Saenko VA, Kanematsu T, Yamashita S (September 2003). "Clinical implication of hot spot BRAF mutation, V599E, in papillary thyroid cancers". J. Clin. Endocrinol. Metab. 88 (9): 4393-7*.*).

In the patient diagnosis it has been found that in 90% of the cases, thymine is substituted with adenine at nucleotide 1799. This will result in an amino acid shift; from valine (V) being substituted to glutamate (E). This will occur at the 600 position, in the activation segment, described in detail previously (Tan YH, Liu Y, Eu KW, Ang PW, Li WQ, Salto-Tellez M, Iacopetta B, Soong R (April 2008). "Detection of BRAF V600E mutation bypyrosequencing". Pathology. 40 (3): 295-8*.).*

The high frequency of activating mutations in tumors and ensuing MAPK pathway addiction make BRAF an attractive therapeutic target, where inhibition of the kinase activity of BRAFV600E and other activated BRAF mutants could provide an effective therapy.

There are drugs that are designed to target cancers with the V600E mutation, such as Vemurafenib, which causes programmed cell death in melanoma cell lines. However, the response efficiency, that was reported recently in a phase 2 study of vemurafenib in patients resulted in that patients with metastatic melanoma, harboring a mutation position at V600-BRAF mutation to E600-BRAF showed a confirmed, independently reviewed overall response rate of 53%. The median with a progressive-free survival of 6.8 months and a median overall survival of 15.9 months (J.A. Sosman, K.B. Kim, L. Schuchter, et al., "Survival in BRAF V600-mutant advanced melanoma treated with vemurafenib" The New England Journal Medicine, 2012, 366; 8 707-714*).*

This shows that even in cases with confirmed E600-BRAF mutation, the response rate is just half of the patients (53%). Thus, for the other 47% that did not respond to the treatment, it would have been not only beneficial but maybe also vital to get an indication if the treatment would work or not, to give time to use other treatments instead. In case of Vemurafenib, it functions by interrupting the B-Raf/MEK step on the B-Raf/MEK/ERK pathway. In effect, the vulnerability to proteasome inhibitors is dependent on persistent BRAF signaling. Therefore we postulate that the actual protein level of V600E BRAF and its ratio to wildtype BRAF is crucial for treatment to work for the tumor. Thus, there is a great demand to investigate the heterogeneity of BRAF protein, WT versus mutated variants, before starting treatment of malignant melanoma with kinase inhibitors. The reason that the specific protein analysis is of mandatory importance, is that the kinase inhibitor drugs are developed towards specific region(s) of three dimensional structure of the protein. In many instances, the affinity interaction and binding constant in-between the drug molecule and the target protein (BRAF), has a given energy threshold level that is optimal for this specific binding, and is determined by the operational window, which means that the cancer drugs are very specific to the correct three dimensional structure. Any alteration of the BRAF protein structure and the drug may not bind correctly, resulting in that a drug molecule may just bind correctly to, and inhibit, a specific fraction of the BRAF proteins in a heterogeneous BRAF protein environment.

The following description focuses on embodiments of the present invention applicable to a mass spectrometry method for measuring the status of BRAF protein sequences, wild type and mutated variants. The quantitation of mutated form(s) of BRAF and the non-mutated BRAF (Wild Type) is determined within this methodology, thereby providing a quantitative read-out of the BRAF protein forms.

Mass spectrometry is a valuable analytical technique because it measures an intrinsic property of any given molecule, its mass, with very high sensitivity. MS can therefore be used to measure a wide range of molecule types and a wide range of sample types/biological materials. In this ionization process, the precursor ion is activated by acceleration into a mass-selective linear ion trap under conditions whereby some of the fragment ions formed are unstable within the trap. After a time delay the stability parameters of the ion trap are changed to allow capture of fragments that were previously unstable. The result is a product ion spectrum that originates from precursor ions with a modified internal energy distribution. It is possible to follow the evolution of the precursor internal energy distribution for many milliseconds after admittance of the precursor ions into the linear ion trap. Time-delayed fragmentation product ion spectra typically display reduced sequential fragmentation products leading to spectra that are more easily interpreted. Several important experimental parameters important to time-delayed fragmentation have been identified and the technique has applications for both small precursor ions and multiply charged molecules.

Tandem mass spectrometry (MS/MS) is at the heart of most of modern mass spectrometric investigations of complex mixtures. The fragmentation involves activation of a precursor ion via collisions with a target gas and may produce charged and neutral fragments. The nature of the fragment ions, as well as their intensities, is often indicative of the structure of the precursor ion and thus can yield useful information for the identification of unknown analytes, as well as providing a useful screening technique for different classes of analytes. Activation via multiple collisions both prolongs the activation time and enables higher energies to be deposited into precursor ions. Higher collision gas pressures also imply higher collision relaxation rates.

Electrospray ionization (ESI) is the most commonly used ionization technique in mass spectrometry which has become an increasingly important technique in the clinical laboratory for quantitative measurements of biomarkers in a complex biological sample. Traditional ESI-MS is a multiple-step process, where the analytes is first introduced into the ionization source of the mass spectrometer, where the analytes are first ionized to acquire positive or negative charges. Then the charged ions travels through the mass analyzer and the ions are sorted and separated according to their mass to charge ratio (m/z value). The separated ions are then passed to the detector system to measure their concentrations, and the results are displayed on a chart called a mass spectrum by a computer system.

When ESI-MS is coupled with a high performance liquid chromatography (HPLC) for analyte fractionation prior to mass spectrometric analysis, HPLC/ESI-MS has become a very powerful technique for analyzing complex biological samples. The instrument will record the spatial distribution of molecular species such as drug compounds, and metabolite drug molecules. Thereafter, suitable image processing software can be used to import data from the mass spectrometer to allow visualization and comparison with the optical image of the sample.

In this invention we pre-fractionate a sample, digested fresh frozen tumors, formalin-fixed and paraffin embedded tumors, as well as bio-fluids (i.e. body fluids), using HPLC with a high pH gradient, or electrostatic chromatographic separation, followed by a second dimension of nano-HPLC interfaced to MS/MS in SRM assay. A quantitative assay of the BRAF peptides reveals the BRAF status of the tumors at the protein level, and a ratio in-between wild type and mutated BRAF proteins is obtained. The BRAF ratio between wild type and mutations will have a critical impact in the response to the treatment of malignant melanoma with kinase inhibitors. Depending on the ratio this will serve as a predictive tool of who will benefit and respond to the treatment.

The serine protease trypsin is most commonly employed for protein digestion as it generates peptides which are highly amenable to MS(/MS) analysis. The protein is cut enzymatically into a limited number of shorter fragments during digestion and these fragments are called peptides and allow for the identification of the protein with their characteristic mass and pattern. However, further studies found that the obtained ratio did not mimic the true ration between WT V600 and mutated V600 BRAF peptides in the cell. In fact, other proteins found in the tumor cells generated fragments with the same sequence as fragments obtained from the wild type of BRAF using trypsin for digestion, thus resulting in a ratio of WT and mutated V600 BRAF that is not correct, which could lead to incorrect treatment decisions. The commonly used enzyme, trypsin, does not generate unique peptide for the wild type form of BRAF. For instance, the generated sequence, IGDFGLATVK (SEQ ID 11) is also found in two other proteins ARAF (Swizz prot. P10398) and RAF1 (Swizz prot. P04049). The BRAF protein reference sequence is found under Swizz prot. P15056.

In the invention it was found that using a serine proteinase that cleaves with high specificity at the C-terminus of glutamic and aspartic acids, here the serine proteinase Glu-C, generated unique peptides both for the wild type and the mutated variants of BRAF. Endoproteinase GluC selectively cleaves peptide bonds C-terminal to glutamic acid residues. Endoproteinase GluC also cleaves at aspartic acid residues, but at a rate 100-300 times slower than at glutamic acid residues, and is therefore referred to as a glutamic-acid-specific Endoprotease.

In one embodiment of the invention, a method for determining the molar ratio between wildtype (WT) BRAF protein and protein variants thereof in a biological sample, wherein the BRAF protein variants are variants mutated in the position corresponding to amino acid position 600 in WT BRAF, said position being occupied by valine in the WT BRAF, comprising the steps of: (a) digesting said sample by using a serine proteinase which specifically cleaves peptide bonds C-terminal to glutamic acid residues or peptide bonds C-terminal to glutamic or aspartic acid residues. This is to obtain a composition comprising a peptide fragment resulting from digestion of the peptides by the proteinase, wherein the mass of said fragment differs between said wild type (WT) B-raf protein and a BRAF protein variant. Thereafter (b) quantitatively assaying the molar amount of the peptide fragment resulting from digestion of wild type (WT) B-raf protein and the molar amount of the peptide fragment resulting from digestion of variants of the wild type (WT) B-raf protein using a mass spectrometry technique. (c) Based on the quantitative assessment calculating the at least one specific ratio between said WT BRAF protein and a variant thereof.

In one embodiment, the BRAF protein variants are BRAF V600E, V600D, V600 R and/or V600K.

In one embodiment, the serine proteinase is glutamyl endopeptidase (Glu-C), such as serine endoproteinase Glu-C (EC 3.4.21.19), such as serine endoproteinase Glu-C belonging to the peptidase family SIB, such as a GluC from Staphylococcus aureus, such as GluC from Staphylococcus aureus V8, such as sequencing grade GluC from Staphylococcus aureus V8 (Promega, Madison, WI). Endoproteinase Glu-C from Staphylococcus aureus strain V8 has an average molecular mass of 29.02 kDa.

It was found that the micro-environment and the catalytic specificity needs to be controlled and are determined by the buffer conditions of the assay. The specificity of glutamic-acid-specific serine proteinase is dependent upon the buffer and pH employed as well as the structure around the potential cleavage site. In ammonium acetate (pH ∼4) or ammonium bicarbonate (pH ∼8) the GluC serine proteinase preferentially cleaves glutamyl bonds. We found that by the use of ammonium bicarbonate generated unique peptides for BRAF, that subsequently result in unique MS/MS fragments within the assay:
SEQ ID 1: DLTVKIGDFGLATVKSRWSGSHQFE (WT),
SEQ ID 2: DLTVKIGDFGLATE (V600E),
SEQ ID 3: DLTVKIGDFGLATKKSRWSGSHQFE (V600K),
SEQ ID 4: DLTVKIGDFGLATRKSRWSGSHQFE (V600R) and
SEQ ID 5: DLTVKIGDFGLATDKSRWSGSHQFE (V600D).

Similarly, we also found that by using phosphate buffer (pH ∼8) the GluC serine proteinase will specifically cleave both glutamyl and aspartyl bonds, thus we were able to generate smaller peptides that were mutated; according to the following BRAF protein sequences
SEQ ID 6: FGLATE (V600E),
SEQ ID 7: GLATD (V600D),
in addition to somewhat longer specific BRAF peptide sequences;
SEQ ID 8: FGLATVKSRWSGSHQFE (WT),
SEQ ID 9: FGLATKKSRWSGSHQFE (V600K), and
SEQ ID 10: FGLATRKSRWSGSHQFE (V600R).

In one embodiment, the digestion step comprises treating said sample with ammonium acetate, ammonium bicarbonate and/or a phosphate buffer.

In one embodiment, the digestion step comprises treating said sample with ammonium acetate at a pH (at 25 °C) between 3.6 to 5.6, such as 3.8 to 4.6, such as about 4.

In one embodiment, the digestion step comprises treating said sample with ammonium bicarbonate at a pH (at 25 °C) between 8.0 to 11.3, such as 8.2 to 9.5, such as about 8.5.

In one embodiment, the digestion step comprises treating said sample with a phosphate buffer at a pH (at 25 °C) between 5.7 to 8, such as 7.0 to 8.0, such as about 7.6.

In one embodiment, the polypetide fragment resulting from digestion of wildtype (WT) B-raf protein used in the quantitative assessment step is a polypeptide according to SEQ ID 1 and/or SEQ ID 8.

In one embodiment, the polypetide fragment resulting from digestion of protein variants of the wildtype (WT) BRAF protein used in the quantitative assessment step is a polypeptide according to SEQ ID 2, SEQ ID 3, SEQ ID 4, SEQ ID 5, SEQ ID 6, SEQ ID 7, SEQ ID 9 and/or SEQ ID 10.

In one embodiment, the polypeptide fragment resulting from digestion of the V600E BRAF protein variant of the wildtype (WT) BRAF protein used in the quantitative assessment step is a polypeptide according to SEQ ID 2 and/or SEQ ID 6.

In one embodiment, the polypeptide fragment resulting from digestion of the V600K BRAF protein variant of the wildtype (WT) BRAF protein used in the quantitative assessment step is a polypeptide according to SEQ ID 3 and/or SEQ ID 9.

In one embodiment, the polypeptide fragment resulting from digestion of the V600R BRAF protein variant of the wildtype (WT) BRAF protein used in the quantitative assessment step is a polypeptide according to SEQ ID 4 and/or SEQ ID 10.

In one embodiment, the polypeptide fragment resulting from digestion of the V600D BRAF protein variant of the wildtype (WT) BRAF protein used in the quantitative assessment step is a polypeptide according to SEQ ID 5 and/or SEQ ID 7.

Thus, specific peptides derived from the sequence of BRAF harboring the WT (V600) and the mutations (V600E, V600D, V600R and V600K) are provided. The peptide sequences and the fragmentation ions for each peptide are used in a Selected Reaction Monitoring assay (SRM) using Liquid Chromatography Mass Spectrometry (LC-MS/MS). In one embodiment, the mass spectrometry technique is HPLC/ESI-MS.

Since proteases not always give rise to 100% complete cleavages of a protein, such alternative cleavage during digestion may result in other possible peptide fragments. However, using mass spectrometry methods for analysis, this can be accounted for, since the peptides resulting from alternative cleavage are still unique and thus allows for the detection and quantification of the WT and/or the mutated variants thereof. Some of the alternative peptides will be observed and/or occur more often than other variants. It was found that alternative cleavage primarily resulted in the following fragments:
SEQ ID 11: LTVKIGDFGLATVKSRWSGSHQFE (WT)
SEQ ID 12: DFGLATVKSRWSGSHQFE (WT)
SEQ ID 13: DLTVKIGDFGLATEKSRWSGSHQFE (V600E)
SEQ ID 14: DFGLATEKSRWSGSHQFE (V600E)
SEQ ID 15: FGLATEKSRWSGSHQFE (V600E)
SEQ ID 16: LTVKIGDFGLATEKSRWSGSHQFE (V600E)
SEQ ID 17: DLTVKIGDFGLATEKSRWSGSHQFE (V600E)
SEQ ID 18: LTVKIGDFGLATEKSRWSGSHQFE (V600E)
SEQ ID 19: DFGLATEKSRWSGSHQFE (V600E)
SEQ ID 20: FGLATEKSRWSGSHQFE (V600E)
SEQ ID 21: LTVKIGDFGLATE (V600E)
SEQ ID 22: DFGLATE (V600E)
SEQ ID 23: DFGLATKKSRWSGSHQFE (V600K)
SEQ ID 24: FGLATKKSRWSGSHQFE (V600K)
SEQ ID 25: LTVKIGDFGLATKKSRWSGSHQFE (V600K)
SEQ ID 26: DLTVKIGDFGLATKSRWSGSHQFE (V600K)
SEQ ID 27: LTVKIGDFGLATKKSRWSGSHQFE (V600K)
SEQ ID 28: DFGLATKKSRWSGSHQFE (V600K)
SEQ ID 29: DFGLATRKSRWSGSHQFE (V600R)
SEQ ID 30: FGLATRKSRWSGSHQFE (V600R)
SEQ ID 31: LTVKIGDFGLATRKSRWSGSHQFE (V600R)
SEQ ID 32: DLTVKIGDFGLATRKSRWSGSHQFE (V600R)
SEQ ID 33: LTVKIGDFGLATRKSRWSGSHQFE (V600R)
SEQ ID 34: DFGLATRKSRWSGSHQFE (V600R)
SEQ ID 35: DFGLATDKSRWSGSHQFE (V600D)
SEQ ID 36: FGLATDKSRWSGSHQFE (V600D)
SEQ ID 37: LTVKIGDFGLATDKSRWSGSHQFE (V600D)
SEQ ID 38: DLTVKIGDFGLATDKSRWSGSHQFE (V600D)
SEQ ID 39: LTVKIGDFGLATDKSRWSGSHQFE (V600D)
SEQ ID 40: DFGLATDKSRWSGSHQFE (V600D)
SEQ ID 41: FGLATDKSRWSGSHQFE (V600D)
SEQ ID 42: DLTVKIGDFGLATD (V600D)
SEQ ID 43: LTVKIGDFGLATD (V600D)
SEQ ID 44: DFGLATD (V600D)
SEQ ID 45: FGLATD (V600D)

In one further disclosure a polypeptide fragment resulting from alternative cleavage during digestion of wildtype (WT) B-raf protein is used in the quantitative assessment step. The polypeptide fragment resulting from such alternative cleavage during digestion of wildtype (WT) B-raf protein may be a polypeptide according to SEQ ID 11 and/or SEQ ID 12.

In one further disclosure a polypeptide fragment resulting from alternative cleavage during digestion of protein variants of the wildtype (WT) B-raf protein is used in the quantitative assessment step. The polypeptide fragment resulting from such alternative cleavage during digestion of protein variants of wildtype (WT) B-raf protein may be a polypeptide according to SEQ ID 13, SEQ ID 14, SEQ ID 15, SEQ ID 16, SEQ ID 17, SEQ ID 18, SEQ ID 19, SEQ ID 20, SEQ ID 21, SEQ ID 22, SEQ ID 23, SEQ ID 24, SEQ ID 25, SEQ ID 26, SEQ ID 27, SEQ ID 28, SEQ ID 29, SEQ ID 30, SEQ ID 31, SEQ ID 32, SEQ ID 33, SEQ ID 34, SEQ ID 35, SEQ ID 36, SEQ ID 37, SEQ ID 38, SEQ ID 39, SEQ ID 40, SEQ ID 41, SEQ ID 42, SEQ ID 43, SEQ ID 44, and/or SEQ ID 45.

In one further disclosure a polypeptide fragment resulting from alternative cleavage during digestion of the V600E protein variant of the wildtype (WT) B-raf protein is used in the quantitative assessment step. The polypeptide fragment resulting from such alternative cleavage during digestion of the V600E protein variant of wildtype (WT) B-raf protein may be a polypeptide according to SEQ ID 13, SEQ ID 14, SEQ ID 15, SEQ ID 16, SEQ ID 17, SEQ ID 18, SEQ ID 19, SEQ ID 20, SEQ ID 21 and/or SEQ ID 22.

In one further disclosure a polypeptide fragment resulting from alternative cleavage during digestion of the V600K protein variant of the wildtype (WT) B-raf protein is used in the quantitative assessment step. The polypeptide fragment resulting from such alternative cleavage during digestion of the V600K protein variant of wildtype (WT) B-raf protein may be a polypeptide according to SEQ ID 23, SEQ ID 24, SEQ ID 25, SEQ ID 26, SEQ ID 27 and/or SEQ ID 28.

In one further disclosure a polypeptide fragment resulting from alternative cleavage during digestion of the V600R protein variant of the wildtype (WT) B-raf protein is used in the quantitative assessment step. The polypeptide fragment resulting from such alternative cleavage during digestion of the V600R protein variant of wildtype (WT) B-raf protein may be a polypeptide according to SEQ ID 29, SEQ ID 30, SEQ ID 31, SEQ ID 32, SEQ ID 33 and/or SEQ ID 34.

In one further disclosure a polypeptide fragment resulting from alternative cleavage during digestion of the V600D protein variant of the wildtype (WT) B-raf protein is used in the quantitative assessment step. The polypeptide fragment resulting from such alternative cleavage during digestion of the V600D protein variant of wildtype (WT) B-raf protein may be a polypeptide according to SEQ ID 35, SEQ ID 36, SEQ ID 37, SEQ ID 38, SEQ ID 39, SEQ ID 40, SEQ ID 41, SEQ ID 42, SEQ ID 43, SEQ ID 44 and/or SEQ ID 45.

In one embodiment, said ratio is between one single BRAF wild type and one single BRAF protein variant. In one alternative embodiment, said ratio is between one single BRAF wild type and at least two BRAF protein variants, such as two, three, four or five BRAF protein variants.

However, using the provided method, more peptides can be derived. The quantitative analysis of the peptides of the BRAF protein is described below in material and methods. This SRM assay can be used to quantitate relative or absolute levels of the WT or mutated peptides of BRAF protein in protein preparation obtained from biological samples, such as digested fresh frozen tumors, formalin-fixed and paraffin embedded tumors, as well as bio-fluids (i.e. body fluids). Tumor tissue can be from a malignant melanoma, a thyroid cancer, a colorectal cancer, a lung cancer, low grade glioma, or an ovarian cancer tumor. Bio-fluids can be blood plasma (EDTA, citrate, heparin), serum, whole blood, buffy coat, platelets, urine and salivia.

Thus in one embodiment of the invention, the sample is a tumor tissue sample or a body fluid. In one further embodiment, the sample is a tumor tissue, the tumor tissue preferably being tissue from a malignant melanoma, a thyroid cancer, a colorectal cancer, a lung cancer, low grade glioma, or an ovarian cancer tumor. In one further embodiment, the sample is a body fluid, preferably the body fluid being blood plasma (EDTA, citrate, heparin), serum, whole blood, buffy coat and platelets.

The current peptides can also be used as the basis for generation and production of new antibody library that will provide novel antibodies for BRAF and its mutated protein forms, for improved sensitivity and specificity.

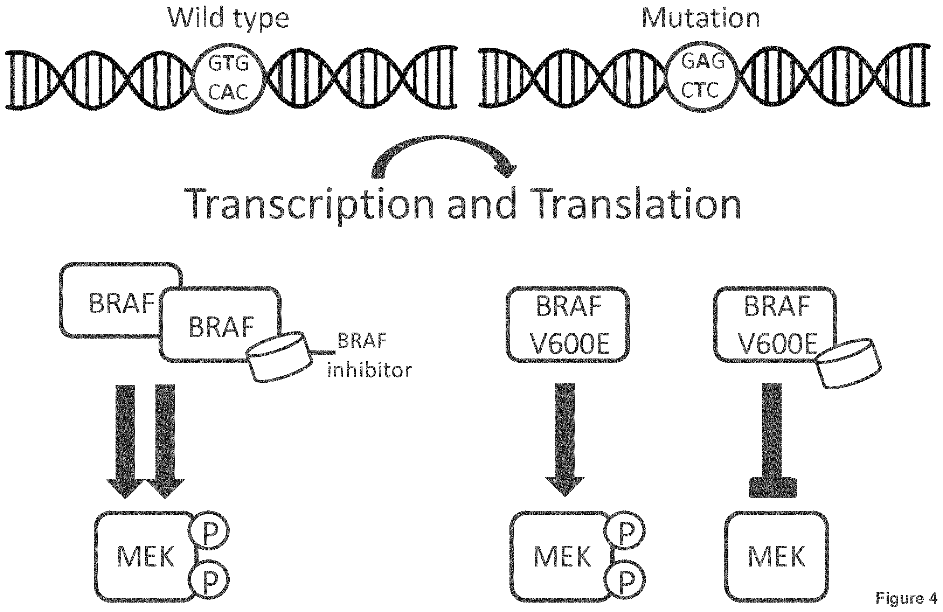

Results from the SRM assay can be used for precise quantitative levels of the mutated and WT variants of the BRAF protein in biological samples (e.g. cancer tissue) and the degree of heterogeneity, WT versus mutated, within biological sample. The information about the heterogeneity of the BRAF peptides within biological samples can allow a physician or other medical professional to more accurately determine appropriate therapy for the patient. Such an SRM-assay can predict which patient is most likely to respond to the kinase inhibitors. The kinase inhibitors, such as Vemurafenib, are developed to act and bind specifically to the 600 amino acid position, where the binding properties are optimized to the mutated 600 position, and not to the Wild Type. This is exemplified by the BRAF mutations at amino acid position 600, illustrated in Fig 4.

In one embodiment of the invention, the mass spectrometry technique is a liquid chromatography interfaced to mass spectrometry technique. In one further embodiment, the mass liquid chromatography interfaced to mass spectrometry technique is HPLC/ESI-MS.

In one embodiment, a subject's susceptibility to a given drug treatment for a BRAF related disease is estimated by the steps of; providing a sample from a subject suffering from a BRAF related disease. The specific molar ratio between WT BRAF protein and variants thereof is determined. The molar specific ratio between WT BRAF protein and variants thereof is compared with a reference value of the specific molar ratio between WT BRAF protein and variant thereof determined from a multitude of samples from subjects known to suffer from said BRAF related disease and to be susceptible to said given drug treatment. Based on said comparison determining the subject's susceptibility to a given drug treatment, wherein a ratio defined by WT BRAF protein to said at least one BRAF protein variant in said sample above the ratio defined by WT BRAF protein to said at least one BRAF protein variant reference value is indicative for an increased susceptibility to a given drug treatment. The reference value is a specific molar ratio between WT BRAF protein and said at least one BRAF protein variant determined from a multitude of samples from subjects known to suffer from said BRAF related disease and to be susceptible to said given drug treatment. The BRAF-related disease is a cancer with BRAF mutations at amino acid position 600, and
the drug treatment is a treatment using a kinase inhibitor, such as Vemurafenib or Sorafenib.

Post-treatment diagnosis is also a highly unmet need, for the melanoma patients, where methodologies determining the mutation status, as well as the ratio outcome to WT on the protein level are a patient read-out test that can provide efficacy of patient status. This can be important in cases where the patient has harbored a tumor comprising cells with heterogeneous mutation profiles, where the treatment only specifically targets part of the tumor cells, resulting in overall tumor shrinkage, but leaving a smaller tumor that does not respond to the drug treatment.

Thus in one embodiment, a subject's susceptibility to a given drug treatment is monitored pre- and post-treatment, wherein a change in the specific molar ratio between WT BRAF protein and variants thereof is determined indicate a changed mutation status of the tumor tissue. Thus, a change in the specific molar ratio may indicate that the treatment has successfully eradicated tumor cells susceptible to given treatment; however, a follow up treatment may be necessary to eradicate other remaining tumor cells. The method of the invention may thus be used to both probe susceptibility to BRAF treatment for the initial tumor cells, as well as the post-treatment remaining tumor cells.

This method also relates to the ratio determination where other mutations might be present in the tumor tissue except for the BRAF 600 V to E mutation, in relation to the WT BRAF. As such, an assay read-out which relates to the measured ratio within this method and innovation also refers to the homozygotic WT versus mutated BRAF patients as well as the heterozygotic WT versus mutated BRAF patients. At the moment, diagnosis with molecular tests, are being used for those inhibitors that are selective for the V to E-BRAF mutation, on the detection of V to E-BRAF in DNA isolated from patient material. This is currently a standard clinical practice, where typically a biopsy material is isolated from the patient and that is fixed in formalin and embedded in paraffin. This test is specific for this setting, and is approved by the FDA. The zygosity within these assays of the V to E-BRAF mutation is not routinely assessed. Thus in one further disclosure the specific molar ratio between WT BRAF protein and variants thereof also refers to the homozygotic WT versus mutated BRAF patients as well as the heterozygotic WT versus mutated BRAF patients.

It has been shown that vemurafenib could enhance the induction of MHC molecules by IFNs in melanoma cells harboring a homozygous BRAF V600E mutation but not heterozygous or wild-type BRAF. The MHC molecules are critical for the interaction between tumor cells and lymphocytes and the enhanced expression of MHC by vemurafenib could promote tumor cell immune recognition. This effect of vemurafenib on the expression of immune system-relevant genes might depend on the zygosity of BRAF V600E, which is not routinely established in melanoma patients

(*Sapkota B, Hill CE, Pollack BP. Vemurafenib enhances MHC induction in BRAF(V600E) homozygous melanoma cells. Oncoimmunology. 2013;2:e22890*). The percentage of homozygous V to E-BRAF mutations in melanoma is unclear, but occurs frequently on a genetic level. It is known that roughly 50% of melanoma patients harboring the V to E-BRAF mutation, as assessed by a conventional sequencing procedure, are homozygous (Rubinstein JC, Sznol M, Pavlick AC, Ariyan S, Cheng E, Bacchiocchi A, et al. Incidence of the V600K mutation among melanoma patients with BRAF mutations, and potential therapeutic response to the specific BRAF inhibitor PLX4032. J Transl Med. 2010;8:67). In addition, the zygosity of V to E-BRAF is able to change over time from heterozygous to homozygous, this was shown in metachronous melanoma metastases from different anatomical locations. The mutational status seemed to be remained unchanged in subsequent melanoma metastases, once the melanoma had reached the metastatic stage. The V to E-BRAF appeared as stable at an early (being present also in benign nevi) mutational event (Sigalotti L, Fratta E, Parisi G, Coral S, Maio M. Stability of BRAF V600E mutation in metastatic melanoma: new insights for therapeutic success? Br J Cancer. 2011;105:327-8)*.*

Thus in one disclosure aspect, an increase over time of a BRAF protein variant in a subject's specific molar ratio between WT BRAF protein and variants thereof, may indicate a change in, or accumulation of, heterozygous and homozygous mutations, a basis for disease dispositioning.

Using the described method of the invention, a medical doctor will be able to make a better treatment decision for targeted treatment for an individual suffering from a BRAF related disease. Thus in one example (not falling under the claims), a method of treatment for a subject with a BRAF related disease, comprises the steps of providing a sample from a subject suffering from a BRAF related disease. The specific molar ratio between WT BRAF protein and variants thereof is determined. The molar specific ratio between WT BRAF protein and variants thereof is compared with a reference value of the specific molar ratio between WT BRAF protein and variant thereof determined from a multitude of samples from subjects known to suffer from said BRAF related disease and to be susceptible to said given drug treatment. Based on said comparison determining the subject's susceptibility to a given drug treatment, wherein a ratio or WT BRAF to said at least one BRAF protein variant in said sample above the ratio or WT BRAF to said at least one BRAF protein variant reference value is indicative for an increased susceptibility to a given drug treatment. If the patient is found susceptibility to a given drug treatment, administer the drug of said drug treatment at a prescribed or defined daily dose (DDD). For vemurafenib, such a prescribed dose may be 500 to 2000 mg, such as 960 mg orally twice a day. For Dabrafenib, such a prescribed dose may be 50 to 300, such as 150 mg twice a day. For sorafenib, such a prescribed dose may be 200 to 800, such as 400 mg twice a day. Treatment duration is usually until efficacy is reached, or unacceptable toxicity occurs. If the patient if found not to be susceptibility to a given drug treatment, this indicates that other treatments must be utilized instead, such as surgery, radiation therapy, chemotherapy, immunotherapy and other treatments beneficial for said BRAF related disease.

In one alternative disclosed aspect, a specific molar ratio between a WT BRAF protein and at least two protein variants thereof is determined. The molar specific ratio between said WT BRAF protein and said at least two variants thereof is compared with a reference value of the specific molar ratio between a WT BRAF protein and at the least two variants thereof determined from a multitude of samples from subjects known to suffer from said BRAF related disease and to be susceptible to said given drug treatment. Based on said comparison determining the subject's susceptibility to a given drug treatment, wherein a ratio or WT BRAF to said at least two BRAF protein variants in said sample above the ratio or WT BRAF to said at least two BRAF protein variants reference value is indicative for an increased susceptibility to a given drug treatment.

If the patient is found susceptible to a given drug treatment, administration of said drug may be in the form of combination treatment. Such combination treatment may comprise combination therapy, such as dabrafenib in combination with trametinib. Such combination treatment may also be administration of the drug the patient is found susceptibility to in combination with other treatments, such as surgery, radiation therapy, chemotherapy and immunotherapy. Thus, the treatment is in the form of a combination treatment. In one further embodiment, such combination treatment is in the form of a combination therapy using two drugs, or in the form of a combination therapy of one drug and one other treatment form, such as surgery, radiation therapy, chemotherapy and immunotherapy.

A molar specific ratio between WT BRAF protein and protein variants thereof as defined by the claims may also be classified as low, medium and high, wherein low includes a ratio of >10:1, medium 10:1 to 1:10 and high 1:>10. Thus in one embodiment, a molar specific ratio between WT BRAF protein and protein variants thereof is classified as low, medium and high, wherein low includes a ratio of >10:1, medium 10:1 to 1:10 and high 1:>10.

In cases where a tumor BRAF variant dominates significantly, treatment is expected to be more effective. In cases where the tumor BRAF wild type dominates significantly, treatment is expected to be less effective. In one disclosed aspect, a method of treatment for a subject with a BRAF related disease, comprises the steps of providing a sample from a subject suffering from a BRAF related disease. The specific molar ratio between WT BRAF protein and variants thereof is determined. In one instance, a low ratio (>10:1) of WT BRAF to said at least one BRAF protein variant in a said sample is indicative of a low susceptibility to a given drug treatment. In one instance, a medium ratio (10:1 to 1:10) of WT BRAF to said at least one BRAF protein variant in a said sample is indicative of at least partial susceptibility to a given drug treatment. It may also be indicative of a heterogeneous tumor harboring both cell populations with WT BRAF and without WT BRAF. In one instance, a high ratio (1:>10) of WT BRAF to said at least one BRAF protein variant in a said sample is indicative of a high susceptibility to a given drug treatment. If the patient is found susceptibility to a given drug treatment, administer the drug of said drug treatment at a prescribed or defined daily dose (DDD).

### Materials and Methods

The following examples are more examples of the invention. Rather, the invention is limited only by the accompanying claims.

### Sample preparation of human tissue

Frozen tissue samples from tumor were sliced into 10 x 10 µm thick sections using a cryostat, performed at -20 C. The sections were lysed in 200 µl of 50 mM ammonium bicarbonate and 6 M urea. The formalin-fixed paraffin embedded tumor tissues were either sectioned using a microtome or cores were excised from the paraffin embedded blocks. The section or the cores were de-paraffinised using EnVision TM FLEX target retrival solution (High pH) (Dako, Glostrup, Denmark and heated for 10 min at 98 C. Samples were centrifuged for 10 min, 14 000 x g at 4 C. The paraffin floating on the surface was removed and the retrieval solution was aspirated. This step was repeated once. To the samples 300 µL 6 M guanidine chloride in 50 mM ammonium bicarbonate was added. The samples were sonicated with a Branson Sonifier 250 (output 4, 10% duty cycle) for 2 minutes followed by centrifugation at 10 000 g for 5 minutes.

The amount of protein in the samples was determined by the BCA method (Pierce, Rockford, IL). A fixed amount (150 µg) of protein were reduced with 10 mM DTT (1 h at 37 °C) and alkylated using 40 mM iodoacetamide (30 min, kept dark at room temperature) followed by buffer exchange with 50 mM ammonium bicarbonate buffer (pH 7.6). The samples were then digested overnight at 37 °C with sequencing grade GluC (Promega, Madison, WI) in a ratio 1:20 w/w (enzyme:protein). The digestion was stopped by adding 30 µL 1% formic acid. The samples were dried using a centrifugal evaporator and resuspended in 150 µL 1% formic acid and centrifuged for 5 min at 10 000 g. The supernatants were stored at -80 °C until further use.

### Peptides

Peptides were supplied by Read Glead Discovery AB (Lund, Sweden) with a purity of more than 95%.

### Generation of transitions list

The transition lists were created in Skyline v3.1.0 software (MacCoss Lab Software, Seattle, WA). Primarily, high numbers of transitions, all possible y-ion series that matches the criteria (from m/z > precursor-2 to last ion-2, precursor m/z exclusion window: 20 Th), were selected for each peptide at both 2+, 3+ and 4+ charge states.

### SRM assay development utilizing TSQ Vantage triple quadrupole MS

The peptide mixture was analyzed by nano LC-MS/MS using a TSQ Vantage triple quadrupole mass spectrometer (Thermo Scientific, Waltham, MA). The TSQ was equipped with an Easy n-LC II pump (Thermo Scientific, Waltham, MA). The samples were injected onto a pre-column Acclaim PepMap100 C8 (5 x 0.3 mm, 5 µm) (Thermo Scientific, Waltham, MA), and following on-line desalting and concentration the peptides were separated on an Acclaim PepMap100 C8 (150 µm x 0.075 mm, 3 µm) (Thermo Scientific, Waltham, MA). Separations were performed in a 35-min linear gradient from 10 to 35% acetonitrile containing 0.1% formic acid; at a flow rate 300 nL/min. The MS analysis was conducted in positive ion mode with the spray voltage and declustering potential were set to 1750 V and 0, respectively. The transfer capillary temperature was set to 270°C and the amplitude of the S-lens was 143. SRM transitions were acquired in Q1 and Q3 operated at unit resolution (0.7 FWHM), the collision gas pressure in Q2 was set to 1.2 mTorr. The cycle time was 3.0 s. Transitions per precursor were selected by manual inspection of the data in Skyline and scheduled transition lists were created for the final assays. The selected transitions were tested in real matrix by spiking the peptide mixtures into human MM tissue digests.

### SRM assay development utilizing QExactive MS

The peptide mixture was analyzed by nano LC-MS/MS using a QExactive mass spectrometer (Thermo Scientific, Waltham, MA). The QExactive was equipped with an Easy n-LC 1000 pump (Thermo Scientific, Waltham, MA). The samples were injected onto a pre-column Acclaim PepMap100 C8 (5 x 0.3 mm, 5 µm) (Thermo Scientific, Waltham, MA), and following on-line desalting and concentration the peptides were separated on an Acclaim PepMap100 C8 (150 µm x 0.075 mm, 3 µm) (Thermo Scientific, Waltham, MA). Separations were performed in a 60-min linear gradient from 5 to 40% acetonitrile containing 0.1% formic acid; at a flow rate 300 nL/min. Full MS scans were acquired in the Orbitrap mass analyzer over m/z 400-1600 range with resolution 70,000 (at m/z 200). The target value was 1.00E+06. An inclusion list for the BRAF peptides was included in the method and the 15 most intense peaks with charge state ≥ 2 were fragmented in the HCD collision cell with normalized collision energy of 30%, and tandem mass spectra were acquired in the Orbitrap mass analyzer with resolution 17,500 at m/z 200. The target value was 1.00E+05. The ion selection threshold was 3.30E+05 counts, and the maximum allowed ion accumulation times were 100 ms for full MS scans and 60 ms for tandem mass spectra. Transitions per precursor were selected by manual inspection of the data in Skyline and scheduled transition lists were created for the final assays. The selected transitions were tested in real matrix by spiking the peptide mixtures into human MM tissue digests.

### Results

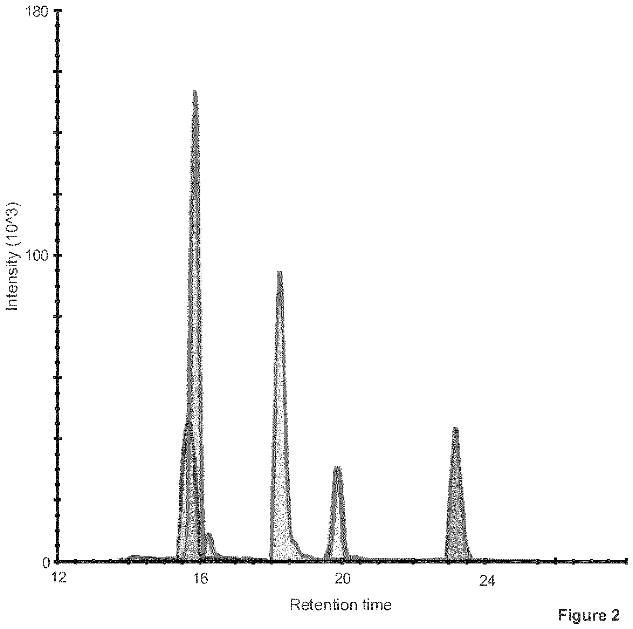

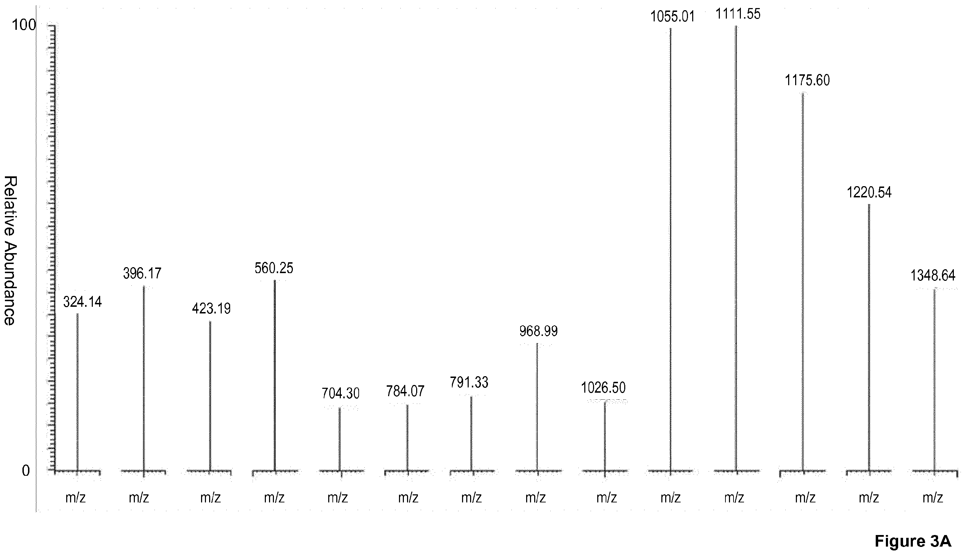

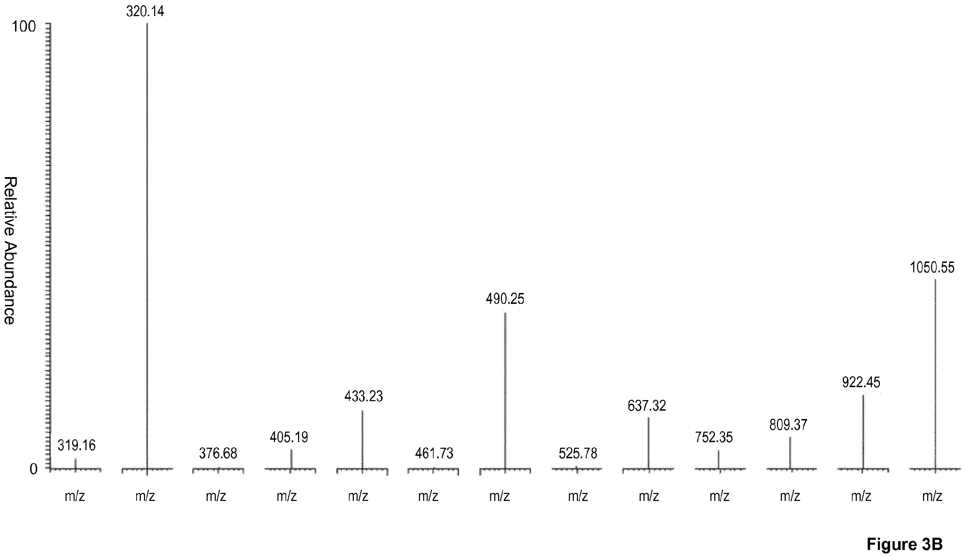

Thus, 10 unique peptide fragments were found for the V600 WT, V600E, V600D, V600R and V600K, part of which are illustrated in figure 2, where SRM signature precursor chromatographic peaks for the GluC generated peptides, the wild type and the four mutated variants of BRAF, in tumor tissue lysate from a fresh frozen tumor sample is shown. Corresponding SRM signatures for the peptides are obtained, which is shown for shown A) wild type and B) V600E in figure 3. Table 1 further summarizes characteristics for the SRM/MRM Signature Chromatographic peaks of these peptides (SEQ ID 1 to SEQ ID 5).

Single amino acid mutagenesis renders a combination of potential protein sequences that functionally will impact the diseases state and staging of disease in patients. This is exemplified by BRAF mutations at amino acid position 600, illustrated in Fig 4.

Below is included a list of GluC peptides sequences and selected SRM transitions for the BRAF peptides:
SEQ ID NO: 1: DLTVKIGDFGLATVKSRWSGSHQFE
   Mono Isotopic Mass: 2779.10
   Precursor m/z: 1389.714 (Charge state: 2)
   Transition m/z: 1548.7554 (y13), 1447.7077 (y12), 1348.6393 (y11), 1220.5443 (y10), 1133.5123 (y9), 977.4112 (y8), 791.3319 (y7), 704.2998 (y6), 647.2784 (y5), 560.2463 (y4), 423.1874 (y3), 1332.2006 (y24), 1275.6586 (y23), 1225.1348 (y22), 1175.6006 (y21), 1111.5531 (y20), 1055.0111 (y19), 1026.5003 (y18), 968.9868 (y17), 895.4526 (y16), 866.9419 (y15), 810.3999 (y14), 774.8813 (y13), 724.3575 (y12), 674.8233 (y11), 610.7758 (y10), 567.2598 (y9), 489.2092 (y8), 396.1696 (y7), 352.6536 (y6), 324.1428 (y5).
   Precursor m/z: 926.8118 (Charge state: 3)
   Transition m/z: 1548.7554 (y13), 1447.7077 (y12), 1348.6393 (y11), 1220.5443 (y10), 1133.5123 (y9), 977.4112 (y8), 791.3319 (y7), 704.2998 (y6), 647.2784 (y5), 560.2463 (y4), 423.1874 (y3), 1332.2006 (y24), 1275.6586 (y23), 1225.1348 (y22), 1175.6006 (y21), 1111.5531 (y20), 1055.0111 (y19), 1026.5003 (y18), 968.9868 (y17), 895.4526 (y16), 866.9419 (y15), 810.3999 (y14), 774.8813 (y13), 724.3575 (y12), 674.8233 (y11), 610.7758 (y10), 567.2598 (y9), 489.2092 (y8), 396.1696 (y7), 352.6536 (y6), 324.1428 (y5), 888.4695 (y24), 850.7748 (y23), 817.0923 (y22), 784.0695 (y21), 741.3711 (y20), 703.6765 (y19), 684.6693 (y18), 646.3270 (y17), 597.3042 (y16), 578.2970 (y15), 540.6023 (y14), 516.9233 (y13), 483.2407 (y12), 450.2179 (y11), 407.5196 (y10), 378.5089 (y9), 326.4752 (y8).
   Precursor m/z: 695.3607 (Charge state: 4)
   Transition m/z: 1548.7554 (y13), 1447.7077 (y12), 1348.6393 (y11), 1220.5443 (y10), 1133.5123 (y9), 977.4112 (y8), 791.3319 (y7), 704.2998 (y6), 647.2784 (y5), 560.2463 (y4), 423.1874 (y3), 1332.2006 (y24), 1275.6586 (y23), 1225.1348 (y22), 1175.6006 (y21), 1111.5531 (y20), 1055.0111 (y19), 1026.5003 (y18), 968.9868 (y17), 895.4526 (y16), 866.9419 (y15), 810.3999 (y14), 774.8813 (y13), 724.3575 (y12), 674.8233 (y11), 610.7758 (y10), 567.2598 (y9), 489.2092 (y8), 396.1696 (y7), 352.6536 (y6), 324.1428 (y5), 888.4695 (y24), 850.7748 (y23), 817.0923 (y22), 784.0695 (y21), 741.3711 (y20), 703.6765 (y19), 684.6693 (y18), 646.3270 (y17), 597.3042 (y16), 578.2970 (y15), 540.6023 (y14), 516.9233 (y13), 483.2407 (y12), 450.2179 (y11), 407.5196 (y10), 378.5089 (y9), 326.4752 (y8).
SEQ ID NO: 2: DLTVKIGDFGLATDKSRWSGSHQFE
   Mono Isotopic Mass: 2795.06
   Precursor m/z: 1397.693 (Charge state: 2)
   Transition m/z: 1564.7139 (y13), 1463.6662 (y12), 1348.6393 (y11), 1220.5443 (y10), 1133.5123 (y9), 977.4112 (y8), 791.3319 (y7), 704.2998 (y6), 647.2784 (y5), 560.2463 (y4), 423.1874 (y3), 1340.1799 (y24), 1283.6379 (y23), 1233.1140 (y22), 1183.5798 (y21), 1119.5323 (y20), 1062.9903 (y19), 1034.4796 (y18), 976.9661 (y17), 903.4319 (y16), 874.9212 (y15), 818.3791 (y14), 782.8606 (y13), 732.3367 (y12), 674.8233 (y11), 610.7758 (y10), 567.2598 (y9), 489.2092 (y8), 396.1696 (y7), 352.6536 (y6), 324.1428 (y5).
   Precursor m/z: 932.1313 (Charge state: 3)
   Transition m/z: 1564.7139 (y13), 1463.6662 (y12), 1348.6393 (y11), 1220.5443 (y10), 1133.5123 (y9), 977.4112 (y8), 791.3319 (y7), 704.2998 (y6), 647.2784 (y5), 560.2463 (y4), 423.1874 (y3), 1340.1799 (y24), 1283.6379 (y23), 1233.1140 (y22), 1183.5798 (y21), 1119.5323 (y20), 1062.9903 (y19), 1034.4796 (y18), 976.9661 (y17), 903.4319 (y16), 874.9212 (y15), 818.3791 (y14), 782.8606 (y13), 732.3367 (y12), 674.8233 (y11), 610.7758 (y10), 567.2598 (y9), 489.2092 (y8), 396.1696 (y7), 352.6536 (y6), 324.1428 (y5), 893.7890 (y24), 856.0943 (y23), 822.4118 (y22), 789.3890 (y21), 746.6907 (y20), 708.9960 (y19), 689.9888 (y18), 651.6465 (y17), 602.6237 (y16), 583.6165 (y15), 545.9219 (y14), 522.2428 (yl3), 488.5603 (y12), 450.2179 (y11), 407.5196 (y10), 378.5089 (y9), 326.4752 (y8).
   Precursor m/z: 699.3503 (Charge state: 4)
   Transition m/z: 1564.7139 (y13), 1463.6662 (y12), 1348.6393 (y11), 1220.5443 (y10), 1133.5123 (y9), 977.4112 (y8), 791.3319 (y7), 704.2998 (y6), 647.2784 (y5), 560.2463 (y4), 423.1874 (y3), 1340.1799 (y24), 1283.6379 (y23), 1233.1140 (y22), 1183.5798 (y21), 1119.5323 (y20), 1062.9903 (y19), 1034.4796 (y18), 976.9661 (y17), 903.4319 (y16), 874.9212 (y15), 818.3791 (y14), 782.8606 (y13), 732.3367 (y12), 674.8233 (y11), 610.7758 (y10), 567.2598 (y9), 489.2092 (y8), 396.1696 (y7), 352.6536 (y6), 324.1428 (y5), 893.7890 (y24), 856.0943 (y23), 822.4118 (y22), 789.3890 (y21), 746.6907 (y20), 708.9960 (y19), 689.9888 (y18), 651.6465 (y17), 602.6237 (y16), 583.6165 (y15), 545.9219 (y14), 522.2428 (yl3), 488.5603 (y12), 450.2179 (y11), 407.5196 (y10), 378.5089 (y9), 326.4752 (y8).
SEQ ID NO: 3: DLTVKIGDFGLATRKSRWSGSHQFE
   Mono Isotopic Mass: 2836.16
   Precursor m/z: 1418.23 (Charge state: 2)
   Transition m/z: 1504.7404 (y12), 1348.6393 (y11), 1220.5443 (y10), 1133.5123 (y9), 977.4112 (y8), 791.3319 (y7), 704.2998 (y6), 647.2784 (y5), 560.2463 (y4), 423.1874 (y3), 1360.7170 (y24), 1304.1750 (y23), 1253.6511 (y22), 1204.1169 (y21), 1140.0694 (y20), 1083.5274 (y19), 1055.0167 (y18), 997.5032 (y17), 923.9690 (y16), 895.4583 (y15), 838.9162 (y14), 803.3977 (y13), 752.8738 (y12), 674.8233 (y11), 610.7758 (y10), 567.2598 (y9), 489.2092 (y8), 396.1696 (y7), 352.6536 (y6), 324.1428 (y5).
   Precursor m/z: 945.8227 (Charge state: 3)
   Transition m/z: 1504.7404 (y12), 1348.6393 (y11), 1220.5443 (y10), 1133.5123 (y9), 977.4112 (y8), 791.3319 (y7), 704.2998 (y6), 647.2784 (y5), 560.2463 (y4), 423.1874 (y3), 1360.7170 (y24), 1304.1750 (y23), 1253.6511 (y22), 1204.1169 (y21), 1140.0694 (y20), 1083.5274 (y19), 1055.0167 (y18), 997.5032 (y17), 923.9690 (y16), 895.4583 (y15), 838.9162 (y14), 803.3977 (y13), 752.8738 (y12), 674.8233 (y11), 610.7758 (y10), 567.2598 (y9), 489.2092 (y8), 396.1696 (y7), 352.6536 (y6), 324.1428 (y5), 907.4804 (y24), 869.7857 (y23), 836.1032 (y22), 803.0804 (y21), 760.3820 (y20), 722.6874 (y19), 703.6802 (y18), 665.3379 (y17), 616.3151 (y16), 597.3079 (y15), 559.6132 (y14), 535.9342 (y13), 502.2516 (y12), 450.2179 (y11), 407.5196 (y10), 378.5089 (y9), 326.4752 (y8).
   Precursor m/z: 709.6189 (Charge state: 4)
   Transition m/z: 1504.7404 (y12), 1348.6393 (y11), 1220.5443 (y10), 1133.5123 (y9), 977.4112 (y8), 791.3319 (y7), 704.2998 (y6), 647.2784 (y5), 560.2463 (y4), 423.1874 (y3), 1360.7170 (y24), 1304.1750 (y23), 1253.6511 (y22), 1204.1169 (y21), 1140.0694 (y20), 1083.5274 (y19), 1055.0167 (y18), 997.5032 (y17), 923.9690 (y16), 895.4583 (y15), 838.9162 (y14), 803.3977 (y13), 752.8738 (y12), 674.8233 (y11), 610.7758 (y10), 567.2598 (y9), 489.2092 (y8), 396.1696 (y7), 352.6536 (y6), 324.1428 (y5), 907.4804 (y24), 869.7857 (y23), 836.1032 (y22), 803.0804 (y21), 760.3820 (y20), 722.6874 (y19), 703.6802 (y18), 665.3379 (y17), 616.3151 (y16), 597.3079 (y15), 559.6132 (y14), 535.9342 (y13), 502.2516 (y12), 450.2179 (y11), 407.5196 (y10), 378.5089 (y9), 326.4752 (y8).
SEQ ID NO: 4: DLTVKIGDFGLATKKSRWSGSHQFE
   Mono Isotopic Mass: 2808.15
   Precursor m/z: 1404.227 (Charge state: 2)
   Transition m/z: 1577.7819 (y13), 1476.7342 (y12), 1348.6393 (y11), 1220.5443 (y10), 1133.5123 (y9), 977.4112 (y8), 791.3319 (y7), 704.2998 (y6), 647.2784 (y5), 560.2463 (y4), 423.1874 (y3), 1346.7139 (y24), 1290.1719 (y23), 1239.6480 (y22), 1190.1138 (y21), 1126.0664 (y20), 1069.5243 (y19), 1041.0136 (y18), 983.5001 (y17), 909.9659 (y16), 881.4552 (y15), 824.9132 (y14), 789.3946 (y13), 738.8708 (y12), 674.8233 (y11), 610.7758 (y10), 567.2598 (y9), 489.2092 (y8), 396.1696 (y7), 352.6536 (y6), 324.1428 (y5).
   Precursor m/z: 936.4874 (Charge state: 3)
   Transition m/z: 1577.7819 (y13), 1476.7342 (y12), 1348.6393 (y11), 1220.5443 (y10), 1133.5123 (y9), 977.4112 (y8), 791.3319 (y7), 704.2998 (y6), 647.2784 (y5), 560.2463 (y4), 423.1874 (y3), 1346.7139 (y24), 1290.1719 (y23), 1239.6480 (y22), 1190.1138 (y21), 1126.0664 (y20), 1069.5243 (y19), 1041.0136 (y18), 983.5001 (y17), 909.9659 (y16), 881.4552 (y15), 824.9132 (y14), 789.3946 (y13), 738.8708 (y12), 674.8233 (y11), 610.7758 (y10), 567.2598 (y9), 489.2092 (y8), 396.1696 (y7), 352.6536 (y6), 324.1428 (y5), 898.1450 (y24), 860.4504 (y23), 826.7678 (y22), 793.7450 (y21), 751.0467 (y20), 713.3520 (y19), 694.3448 (y18), 656.0025 (y17), 606.9797 (y16), 587.9725 (y15), 550.2779 (y14), 526.5988 (y13), 492.9163 (y12), 450.2179 (y11), 407.5196 (y10), 378.5089 (y9), 326.4752 (y8).
   Precursor m/z: 702.6173 (Charge state: 4)
   Transition m/z: 1577.7819 (y13), 1476.7342 (y12), 1348.6393 (y11), 1220.5443 (y10), 1133.5123 (y9), 977.4112 (y8), 791.3319 (y7), 704.2998 (y6), 647.2784 (y5), 560.2463 (y4), 423.1874 (y3), 1346.7139 (y24), 1290.1719 (y23), 1239.6480 (y22), 1190.1138 (y21), 1126.0664 (y20), 1069.5243 (y19), 1041.0136 (y18), 983.5001 (y17), 909.9659 (y16), 881.4552 (y15), 824.9132 (y14), 789.3946 (y13), 738.8708 (y12), 674.8233 (y11), 610.7758 (y10), 567.2598 (y9), 489.2092 (y8), 396.1696 (y7), 352.6536 (y6), 324.1428 (y5), 898.1450 (y24), 860.4504 (y23), 826.7678 (y22), 793.7450 (y21), 751.0467 (y20), 713.3520 (y19), 694.3448 (y18), 656.0025 (y17), 606.9797 (y16), 587.9725 (y15), 550.2779 (y14), 526.5988 (y13), 492.9163 (y12), 450.2179 (y11), 407.5196 (y10), 378.5089 (y9), 326.4752 (y8).
SEQ ID NO: 5: DLTVKIGDFGLATE
   Mono Isotopic Mass: 1478.66
   Precursor m/z: 739.8905 (Charge state: 2)
   Transition m/z: 1363.7468 (y13), 1250.6627 (y12), 1149.6150 (y11), 1050.5466 (y10), 922.4516 (y9), 809.3676 (y8), 752.3461 (y7), 637.3192 (y6), 490.2508 (y5), 433.2293 (y4), 320.1452 (y3), 682.3770 (y13), 625.8350 (y12), 575.3111 (y11), 525.7769 (y10), 461.7295 (y9), 405.1874 (y8), 376.6767 (y7), 319.1632 (y6).
   Precursor m/z: 493.5961 (Charge state: 3)
   Transition m/z: 1363.7468 (y13), 1250.6627 (y12), 1149.6150 (y11), 1050.5466 (y10), 922.4516 (y9), 809.3676 (y8), 752.3461 (y7), 637.3192 (y6), 490.2508 (y5), 433.2293 (y4), 320.1452 (y3), 682.3770 (y13), 625.8350 (y12), 575.3111 (y11), 525.7769 (y10), 461.7295 (y9), 405.1874 (y8), 376.6767 (y7), 319.1632 (y6), 455.2538 (y13), 417.5591 (y12), 383.8765 (y11), 350.8537 (y10), 308.1554 (y9).
   Precursor m/z: 370.4489 (Charge state: 4)
   Transition m/z: 1363.7468 (y13), 1250.6627 (y12), 1149.6150 (y11), 1050.5466 (y10), 922.4516 (y9), 809.3676 (y8), 752.3461 (y7), 637.3192 (y6), 490.2508 (y5), 433.2293 (y4), 320.1452 (y3), 682.3770 (y13), 625.8350 (y12), 575.3111 (y11), 525.7769 (y10), 461.7295 (y9), 405.1874 (y8), 376.6767 (y7), 319.1632 (y6), 455.2538 (y13), 417.5591 (y12), 383.8765 (y11), 350.8537 (y10), 308.1554 (y9).

In the claims, the term "comprises/comprising" does not exclude the presence of other elements or steps. Furthermore, although individually listed, a plurality of means, elements or method steps may be implemented by e.g. a single unit or processor. In addition, singular references do not exclude a plurality. The terms "a", "an", "first", "second" etc do not preclude a plurality.

## Claims

1. A method for determining the molar ratio between wild type (WT) BRAF (v-raf murine sarcoma viral oncogene homolog B1) protein and protein variants thereof in a biological sample, wherein the BRAF protein variants are variants mutated in the position corresponding to amino acid position 600 in WT BRAF, said position being occupied by valine in the WT BRAF, comprising the steps of;
a) digesting said sample by using a serine proteinase which specifically cleaves peptide bonds C-terminal to glutamic acid residues or peptide bonds C-terminal to glutamic or aspartic acid residues, to obtain a composition comprising a peptide fragment resulting from digestion of the peptides by the proteinase, wherein the mass of said fragment differs between said wild type (WT) B-raf protein and a BRAF protein variant;
b) quantitatively assaying the molar amount of a peptide fragment resulting from digestion of wild type (WT) B-raf protein and the molar amount of a peptide fragment resulting from digestion of variants of the wild type (WT) B-raf protein using a mass spectrometry technique; and
c) based on the quantitative assessment calculating the at least one specific ratio between said WT BRAF protein and a variant thereof.

2. The method of claim 1, wherein the BRAF protein variants are BRAF V600E, V600D, V600R, and/or V600K.

3. The method of any of claims 1 to 2, wherein the serine proteinase is glutamyl endopeptidase (Glu-C).

4. The method of any of claims 1 and 3, wherein said digestion step comprises treating said sample with ammonium acetate, ammonium bicarbonate and/or a phosphate buffer.

5. The method of any of claims 1 to 4, wherein the sample is a tumor tissue sample or a body fluid.

6. The method of any of claims 1 to 5, wherein the sample is tumor tissue, the tumor tissue being tissue from a malignant melanoma, a thyroid cancer, a colorectal cancer, a lung cancer, brain tumor cancer, low grade glioma, or an ovarian cancer tumor.

7. The method of any of claims 1 to 6, wherein the polypetide fragment resulting from digestion of wildtype (WT) B-raf protein used in the quantitative assessment step is a polypeptide according to SEQ ID 1 and/or SEQ ID 8.

8. The method of claim 2, wherein the polypetide fragment resulting from digestion of protein variants of the wild type (WT) B-raf protein measured in the quantitative assessment step is a polypeptide according to to SEQ ID 2, SEQ ID 3, SEQ ID 4, SEQ ID 5, SEQ ID 6, SEQ ID 7, SEQ ID 9, and/or SEQ ID 10.

9. The method of any of claims 1 to 6, wherein the mass spectrometry technique is a liquid chromatography interfaced to mass spectrometry technique.

10. The method of claim 9, wherein the mass liquid chromatography interfaced to mass spectrometry technique is HPLC/ESI-MS.

11. Method for estimating a subject's susceptibility to a given drug treatment for a BRAF related disease, comprising the steps of:
(i) providing a sample from a subject suffering from a BRAF related disease; and determining the specific molar ratio between WT BRAF protein and variants thereof by a method according to any of claims 1 to 10;
(ii) comparing the molar specific ratio between WT BRAF protein and variants thereof, with a reference value of the specific molar ratio between WT BRAF protein and variant thereof determined from a multitude of samples from subjects known to suffer from said BRAF related disease and to be susceptible to said given drug treatment;
(iii) based on said comparison determining the subject's susceptibility to a given drug treatment,
wherein a ratio defined by WT BRAF protein to said at least one BRAF protein variant in said sample above the ratio defined by WT BRAF protein to said at least one BRAF protein variant reference value is indicative for an increased susceptibility to a given drug treatment,
the reference value is a specific molar ratio between WT BRAF protein and said at least one BRAF protein variant determined from a multitude of samples from subjects known to suffer from said BRAF related disease and to be susceptible to said given drug treatment,
the BRAF-related disease is a cancer with BRAF mutations at amino acid position 600, and
said drug treatment is a treatment using a kinase inhibitor, such as Vemurafenib, Dabrafenib or Sorafenib.

12. The method according to claim 11, wherein a subject's susceptibility to a given drug treatment is monitored pre- and post-treatment, wherein a change in the specific molar ratio between WT BRAF protein and variants thereof between pre- and post-operation, indicates a changed mutation status of the tumor tissue.

## Patentansprüche

1. Verfahren zur Bestimmung des molaren Verhältnisses zwischen Wildtyp (WT) BRAF (v-raf murine sarcoma viral oncogene homolog B1) -Protein und Proteinvarianten davon in einer biologischen Probe, wobei die BRAF-Proteinvarianten Varianten sind, die in der Position mutiert sind, die der Aminosäureposition 600 in WT BRAF entspricht, wobei die Position durch Valin im WT BRAF besetzt ist, umfassend die Schritte von:
a) Verdauen der Probe unter Verwendung einer Serinproteinase, die spezifisch Peptidbindungen C-terminal von Glutaminsäureresten oder Peptidbindungen C-terminal von Glutamin- oder Asparaginsäureresten spaltet, um eine Zusammensetzung zu erhalten, die ein Peptidfragment umfasst, das aus dem Verdau der Peptide durch die Proteinase resultiert, wobei sich die Masse des Fragments zwischen dem Wildtyp (WT)-B-raf-Protein und einer BRAF-Proteinvariante unterscheidet;
b) quantitatives Bestimmen der molaren Menge eines Peptidfragments, das aus der Verdauung des Wildtyp (WT)-B-raf-Proteins resultiert, und der molaren Menge eines Peptidfragments, das aus der Verdauung von Varianten des Wildtyp (WT)-B-raf-Proteins resultiert, unter Verwendung einer Massenspektrometrie-Technik; und,
c) auf der Grundlage der quantitativen Bestimmung, Berechnen des mindestens einen spezifischen Verhältnisses zwischen dem WT BRAF-Protein und einer Variante davon.

2. Verfahren nach Anspruch 1, wobei die BRAF-Proteinvarianten BRAF V600E, V600D, V600R und/oder V600K sind.

3. Verfahren nach einem der Ansprüche 1 bis 2, wobei die Serinproteinase Glutamyl-Endopeptidase (Glu-C) ist.

4. Verfahren nach einem der Ansprüche 1 und 3, wobei der Verdauungsschritt die Behandlung der Probe mit Ammoniumacetat, Ammoniumbicarbonat und/oder einem Phosphatpuffer umfasst.

5. Verfahren nach einem der Ansprüche 1 bis 4, wobei die Probe eine Tumorgewebeprobe oder eine Körperflüssigkeit ist.

6. Verfahren nach einem der Ansprüche 1 bis 5, wobei die Probe Tumorgewebe ist, wobei das Tumorgewebe Gewebe aus einem malignen Melanom, einem Schilddrüsenkrebs, einem kolorektalen Krebs, einem Lungenkrebs, einem Gehirntumor, einem niedriggradigen Gliom oder einem Eierstockkrebstumor ist.

7. Verfahren nach einem der Ansprüche 1 bis 6, wobei das Polypetidfragment, das aus der Verdauung des Wildtyp (WT)-B-raf-Proteins resultiert und in dem quantitativen Bestimmungsschritt verwendet wird, ein Polypeptid gemäß SEQ Nr. 1 und/oder SEQ Nr. 8 ist.

8. Verfahren nach Anspruch 2, wobei das Polypetidfragment, das aus dem Verdau von Proteinvarianten des Wildtyp-(WT)-B-raf-Proteins resultiert, das in dem quantitativen Bestimmungsschritt gemessen wird, ein Polypeptid gemäß SEQ Nr. 2, SEQ Nr. 3, SEQ Nr. 4, SEQ Nr. 5, SEQ Nr. 6, SEQ NR. 7, SEQ Nr. 9 und/oder SEQ Nr. 10 ist.

9. Verfahren nach einem der Ansprüche 1 bis 6, wobei die Massenspektrometrie-Technik eine Flüssigchromatographie mit Schnittstelle zur Massenspektrometrie-Technik ist.

10. Verfahren nach Anspruch 9, wobei die Technik der Flüssigchromatographie mit Schnittstelle zur Massenspektrometrie HPLC/ESI-MS ist.

11. Verfahren zur Beurteilung der Suszeptibilität eines Subjekts auf eine gegebene Arzneimittelbehandlung für eine BRAF-bezogene Erkrankung, umfassend die folgenden Schritte:
(i) Bereitstellen einer Probe von einem Subjekt, das an einer BRAF-bezogenen Erkrankung leidet; und Bestimmen des spezifischen molaren Verhältnisses zwischen WT-BRAF-Protein und Varianten davon durch ein Verfahren nach einem der Ansprüche 1 bis 10;
(ii) Vergleichen des spezifischen molaren Verhältnisses zwischen WT BRAF-Protein und Varianten davon mit einem Referenzwert des spezifischen molaren Verhältnisses zwischen WT BRAF-Protein und Varianten davon, der aus einer Vielzahl von Proben von Subjekten bestimmt wurde, von denen bekannt ist, dass sie an der BRAF-bezogenen Erkrankung leiden und für die gegebene Arzneimittelbehandlung empfänglich sind;
(iii) auf der Grundlage des Vergleichs, Bestimmen der Suszeptibilität des Subjekts auf eine gegebene Arzneimittelbehandlung,
wobei ein durch das WT-BRAF-Protein definiertes Verhältnis zu der mindestens einen BRAF-Proteinvariante in der Probe oberhalb des durch das WT-BRAF-Protein definierten Verhältnisses zu dem Referenzwert der mindestens einen BRAF-Proteinvariante indikativ für eine erhöhte Suszeptibilität auf eine gegebene Arzneimittelbehandlung ist,
der Referenzwert ein spezifisches molares Verhältnis zwischen WT-BRAF-Protein und der mindestens einen BRAF-Proteinvariante ist, der aus einer Vielzahl von Proben von Subjekten bestimmt wurde, von denen bekannt ist, dass sie an der BRAF-bezogenen Erkrankung leiden und für die gegebene Arzneimittelbehandlung empfänglich sind,
die BRAF-bezogene Erkrankung ein Krebs mit BRAF-Mutationen an der Aminosäureposition 600 ist, und
die Arzneimittelbehandlung eine Behandlung unter Verwendung eines Kinaseinhibitors, wie Vemurafenib, Dabrafenib oder Sorafenib, ist.

12. Verfahren nach Anspruch 11, wobei die Suszeptibilität eines Subjekts auf eine gegebene Arzneimittelbehandlung vor und nach der Behandlung überwacht wird, wobei eine Änderung des spezifischen molaren Verhältnisses zwischen WT-BRAF-Protein und Varianten davon zwischen der Vor- und Nachbehandlung indiaktiv für einen veränderten Mutationsstatus des Tumorgewebes ist.

## Revendications

1. Procédé de détermination du rapport molaire entre une protéine BRAF (homologue B1 de l'oncogène viral de sarcome murin v-raf) de type sauvage (WT) et des variants protéiques de celle-ci dans un échantillon biologique, dans lequel les variants protéiques de BRAF sont des variants présentant une mutation à la position correspondant à la position d'acide aminé 600 dans BRAF WT, ladite position étant occupée par une valine dans BRAF WT, comprenant les étapes consistant à :
a) digérer ledit échantillon au moyen d'une sérine protéinase qui clive spécifiquement les liaisons peptidiques C-terminales aux résidus acide glutamique ou les liaisons peptidiques C-terminales aux résidus acide glutamique ou aspartique, pour obtenir une composition comprenant un fragment peptidique résultant de la digestion des peptides par la protéinase, dans lequel la masse dudit fragment diffère entre ladite protéine B-raf de type sauvage (WT) et un variant protéique de BRAF ;
b) doser quantitativement la quantité molaire d'un fragment peptidique résultant de la digestion de la protéine B-raf de type sauvage (WT) et la quantité molaire d'un fragment peptidique résultant de la digestion de variants de la protéine B-raf de type sauvage (WT) au moyen d'une technique de spectrométrie de masse ; et
c) sur la base de l'évaluation quantitative, calculer le au moins un rapport spécifique entre ladite protéine BRAF WT et un variant de celle-ci.

2. Procédé selon la revendication 1, dans lequel les variants protéiques de BRAF sont BRAF V600E, V600D, V600R et/ou V600K.

3. Procédé selon l'une quelconque des revendications 1 à 2, dans lequel la sérine protéinase est une glutamyl endopeptidase (Glu-C).

4. Procédé selon l'une quelconque des revendications 1 et 3, dans lequel ladite étape de digestion comprend le traitement dudit échantillon avec de l'acétate d'ammonium, du bicarbonate d'ammonium et/ou un tampon au phosphate.

5. Procédé selon l'une quelconque des revendications 1 à 4, dans lequel l'échantillon est un échantillon de tissu tumoral ou un fluide corporel.

6. Procédé selon l'une quelconque des revendications 1 à 5, dans lequel l'échantillon est un tissu tumoral, le tissu tumoral étant un tissu provenant d'un mélanome malin, d'un cancer de la thyroïde, d'un cancer colorectal, d'un cancer du poumon, d'un cancer de tumeur cérébrale, d'un gliome de bas grade ou d'une tumeur cancéreuse ovarienne.

7. Procédé selon l'une quelconque des revendications 1 à 6, dans lequel le fragment polypeptidique résultant de la digestion de la protéine B-raf de type sauvage (WT) utilisé dans l'étape d'évaluation quantitative est un polypeptide selon SEQ ID 1 et/ou SEQ ID 8.

8. Procédé selon la revendication 2, dans lequel le fragment polypeptidique résultant de la digestion des variants protéiques de la protéine B-raf de type sauvage (WT) mesuré dans l'étape d'évaluation quantitative est un polypeptide selon SEQ ID 2, SEQ ID 3, SEQ ID 4, SEQ ID 5, SEQ ID 6, SEQ ID 7, SEQ ID 9 et/ou SEQ ID 10.

9. Procédé selon l'une quelconque des revendications 1 à 6, dans lequel la technique de spectrométrie de masse est une chromatographie en phase liquide interfacée avec une technique de spectrométrie de masse.

10. Procédé selon la revendication 9, dans lequel la chromatographie de masse en phase liquide interfacée avec une technique de spectrométrie de masse est une CLHP/ESI-SM.

11. Procédé d'estimation de la sensibilité d'un sujet à un traitement médicamenteux donné pour une maladie associée à BRAF, comprenant les étapes consistant à :
(i) fournir un échantillon provenant d'un sujet souffrant d'une maladie associée à BRAF ; et déterminer le rapport molaire spécifique entre la protéine BRAF WT et les variants de celle-ci par un procédé selon l'une quelconque des revendications 1 à 10 ;
(ii) comparer le rapport molaire spécifique entre la protéine BRAF WT et les variants de celle-ci, à une valeur de référence du rapport molaire spécifique entre la protéine BRAF WT et un variant de celle-ci déterminé à partir d'une multitude d'échantillons provenant de sujets connus pour souffrir de ladite maladie associée à BRAF et pour être sensible audit traitement médicamenteux donné ;
(iii) sur la base de ladite comparaison, déterminer la sensibilité du sujet à un traitement médicamenteux donné,
dans lequel un rapport entre la protéine BRAF WT et ledit au moins un variant protéique de BRAF dans ledit échantillon supérieur à la valeur de référence du rapport entre la protéine BRAF WT et ledit au moins un variant protéique de BRAF indique une sensibilité accrue à un traitement médicamenteux donné,
la valeur de référence est un rapport molaire spécifique entre la protéine BRAF WT et ledit au moins un variant protéine de BRAF déterminé à partir d'une multitude d'échantillons provenant de sujets connus pour souffrir de ladite maladie associée à BRAF et pour être sensible audit traitement médicamenteux donné,
la maladie associée à BRAF est un cancer avec des mutations dans BRAF à la position d'acide aminé 600, et
ledit traitement médicamenteux est un traitement utilisant un inhibiteur de kinase, tel que le vémurafénib, le dabrafénib ou le sorafénib.

12. Procédé selon la revendication 11, dans lequel la sensibilité d'un sujet à un traitement médicamenteux donné est surveillée avant et après le traitement, dans lequel un changement du rapport molaire spécifique entre la protéine BRAF WT et des variants de celle-ci entre une pré- et une post-opération, indique une modification du statut mutationnel du tissu tumoral.
